# EUROPEAN PATENT APPLICATION

(11) **EP 4 357 359 A1**
(43) Date of publication of application: **24.04.2024**
(21) Application number: 23205005.4
(22) Date of filing: 20.10.2023
(51) Int. Cl.: C07K 14/705, A61K 31/00, A61K 33/00, A61K 38/00, A61P 1/00, A61P 25/00, A61P 27/02, C12N 15/00, C12N 15/52, C12Q 1/68

(54) **G-PROTEIN-GATED-K+ CHANNEL-MEDIATED ENHANCEMENTS IN LIGHT SENSITIVITY IN ROD-CONE DYSTROPHY (RCD)**

(30) Priority: 20.10.2022 EP 22306596
(71) Applicant: Sparingvision, 75008 Paris (FR); Ruhr-Universität Bochum, 44801 Bochum (DE); Institut National de la Santé et de la Recherche Médicale, 75013 Paris (FR); Centre National de la Recherche Scientifique, 75016 Paris (FR); SORBONNE UNIVERSITE, 75006 Paris (FR)
(72) Inventor: DALKARA, Deniz, 94140 ALFORTVILLE (FR); KHABOU, Hanen, 93160 NOISY LE GRAND (FR); MÜCHER, Brix, 44801 BOCHUM (FR); HERLITZE, Stefan, 44797 BOCHUM (FR)
(74) Representative: Plasseraud IP

(57) **Abstract**

The present invention concerns a new gene therapy approach to increase light-sensitivity in degenerating cones in advanced stages of rod-cone dystrophy (RCD) mediated by G-protein-gated inwardly rectifying potassium channel (GIRK), in particular GIRK4 S143T, activated by G proteins recruited by cone opsin expressed in degenerating cones.

## Description

### Technical field of the invention

The present invention concerns a new gene therapy approach to increase light-sensitivity in degenerating cones in intermediate to advanced stages of rod-cone dystrophy (RCD) mediated by G-protein-gated inwardly rectifying potassium channel (GIRK), in particular GIRK4 S143T, activated by G proteins recruited by cone opsin expressed in degenerating cones.

In the description below, references in square brackets ([]) refer to the list of references at the end of the text.

### State of the art

Retina is the light sensitive tissue of the eye composed of three layers of neurons interconnected by synapses. The primary neurons of the retina are the light-sensing photoreceptors (PR), which are of two types: the rods for night vision and the cones for daylight vision. Cone-mediated vision is mostly supported by the fovea and is responsible for high acuity central vision most valuable to our daily visual tasks (Sinha et al., 2017) [1]. The light sensitive G protein coupled receptors that link photon capture to intracellular signaling leading to membrane hyperpolarization in photoreceptors are called opsins (Yau and Hardie, 2009) [2]. There is one type of rod opsin found in rods and three types of cone opsins - responsible for trichromatic vision - in the primate retina. The structural and phototransduction cascades properties are similar between these opsins.

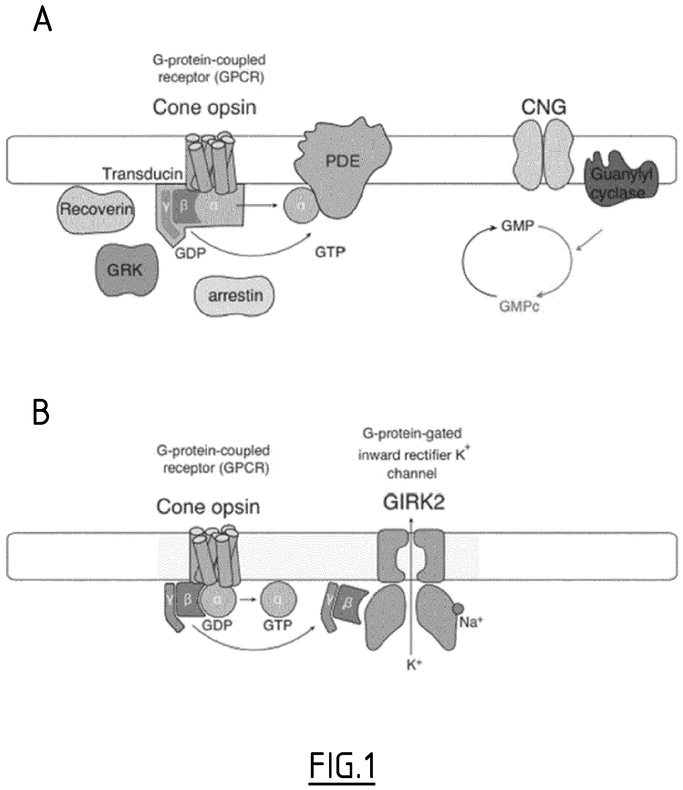

The phototransduction cascade is composed of several proteins that are concentrated in the photoreceptor outer segments in normal retinas (Figure 1A). The role of the photoreceptor is to sense light via this phototransduction cascade and induce an electrical signal that is then processed and transmitted towards downstream neurons (Ebrey and Koutalos, 2001) [3].

The absorption of a photon activates the opsin composed of two parts: the protein part, and the light absorbing part, which is the retinal - a derivative of vitamin A. The latter isomerizes from 11-cis-retinal (dark adapted state) into all-trans-retinal configuration (light adapted state). As a result, the opsin becomes catalytically active recruiting the G protein transducin. The α-subunit of transducin is activated by the replacement of GDP by GTP. After, the α-subunit dissociates from the βγ-subunits to activate the membrane-associated phosphodiesterase 6 (PDE) by binding its two inhibitory y subunits. The activated PDE hydrolyses cGMP into GMP. The reduction of cGMP clones the nucleotide-gated channels (CNG) and this stops cation entry, resulting in PR hyperpolarization and reduction in glutamate release by the photoreceptor (Larhammar et al., 2009) [4].

In order to respond to another photon, this phototransduction cascade is deactivated by two mechanisms: (i) the transducin inactivates itself by hydrolyzing the bound GTP and (ii) the rhodopsin kinase (GRK) phosphorylates the opsin that interacts with the regulatory protein arrestin, leading to opsin inactivation. Retinal is then recycled by the retinal pigment epithelium (RPE) and Müller glial cells. Each and every protein of this cascade plays an important role in converting the light signal into an electrical signal conveyed to the second and third order neurons (Maeda et al., 2003) [5].

Inherited retinal degenerations are mostly due to mutations in photoreceptor or RPE cells leading to the degeneration of the rods, followed by cone outer segment degeneration and dysfunction eventually leading to blindness (Buch et al., 2004) [6]. Among them, rod-cone dystrophy (RCD) represents the largest category where genetic causes are highly heterogeneous. More than 60 different genes expressed in rod photoreceptors or the retinal pigment epithelium are involved (Wright et al., 2010) [7]. The first gene that has been linked to RCD is the rhodopsin gene *RHO* that counts for 25% of RCD autosomal dominant cases. Many other causative genes have also been identified: the cGMP-PDE subunit gene and the cyclic GMP gated channel protein α subunit gene. Although there are many causative genes, the resulting RCD phenotype is the same across different mutations (Ferrari et al., 2011) [8]. The common RCD phenotype is characterized by the progressive rod degeneration, causing night blindness, and followed by progressive peripheral cone degeneration, causing "tunnel vision", mediated entirely by the remaining foveal cones then eventually resulting in complete blindness in the latest stages of disease. Usually, when patients are diagnosed with RCD, they already show night blindness, meaning their rods have degenerated. However, the cones remain until the late stages of the disease; particularly in the foveal region responsible for high acuity and leading to tunnel vision in intermediate stages (Li et al., 1995) [9]. In later stages of the disease, these cones lose their outer segment structures leading to complete blindness before the complete loss of the cone soma and pedicle (Li et al., 1995) [9].

In order to preserve vision in these patients presenting degenerating cones or light sensitive cone cell bodies, one innovative strategy is retinal gene therapy, which broadly refers to the transfer of a therapeutic gene into retinal cells to mediate a therapeutic effect (Bennett, 2017) [10]. Although the first successful clinical trials of gene therapy have focused on gene replacement, where a gene carrying a recessive mutation is replaced by a functional cDNA copy, this strategy is limited because it cannot be used for the majority of retinal degenerations (Bennett, 2017) [10]. For example, in RCD, the huge variability of mutations makes it difficult to develop gene therapy to each specific mutation. Moreover, dominant mutations cannot be treated using this approach. Furthermore, in 50% of cases, the causative mutation is not elucidated or the rod photoreceptors bearing the most frequent mutations are already lost (Dalkara et al., 2015) [11]. For these reasons, mutation-independent gene therapies that can be applied beyond the loss of rods must be developed to treat a large number of patients without knowledge of the mutant gene.

Taking this goal into account, previous studies used the ectopic expression of microbial opsins, like channelrhodopsin in bipolar cells or halorhodopsin in cones PRs, to modulate membrane potential and induce a depolarization or hyperpolarization respectively (Busskamp et al., 2012; Dalkara and Sahel, 2014; Scholl et al., 2016) [12-14]. Thus, in RCD, optogenetics can be used as a therapeutic strategy to restore vision in blind retinas (Baker and Flannery, 2008) [15]. There are many parameters to consider: (i) the choice of cell target to make the most out of the retinal circuitry sending the most interpretable signal to the brain (ii) the choice of the appropriate optogenetic tool to obtain a close to natural electrophysiological response in these target cells. Concerning the second point, the relatively weak capacity of microbial opsins has been a major limitation: potential immunogenicity of using an opsin from prokaryotic species, high intensities required due to the lack of signal amplification by these directly light gated channels and pumps originating from single cell microorganisms, as in the case of halorhodopsin (Baker and Flannery, 2008) [15], (Cehajic-Kapetanovic et al., 2015; Gaub et al., 2015; Van Gelder and Kaur, 2015; van Wyk et al., 2015) [16-19]. Unlike rhodopsins or cone opsins, microbial opsins are not able to activate G protein coupled cascades such as the phototransduction cascade present in healthy retinas. One of the possible ways to go beyond the limits of microbial opsins is the use of animal opsins, which are all G protein coupled receptors. However, all work in this field has so far been focused on inner retinal neurons (Berry et al., 2019; Cehajic-Kapetanovic et al., 2015; De Silva et al., 2017; Gaub et al., 2015; Lin et al., 2008; van Wyk et al., 2015) [20, 16, 21, 17, 22, 19].

In a previous study, it has been shown that light-activation of animal cone opsins can stimulate the G_{i/o} signaling pathways in human kidney cells and in neuronal cells *in vitro* and *in vivo* (Masseck et al., 2014) [23]. This pathway is involved in fast dampening of neuronal activity and inhibition of intrinsic ion channels. However, it has also been shown that animal cone opsins can directly modulate the G protein-gated inwardly rectifying potassium channels (GIRK) upon co-expression in any given cell (Berry et al., 2019; Masseck et al., 2014) [20, 23]. GIRK channels are composed of two subunits. There are four types of subunits: GIRK1 to 4. GIRK1, 4 and 3 cannot form homotetramers; they have to be associated with another subunit to be functional (Mark and Herlitze, 2000) [24]. Conversely, GIRK2 alone can form homotetramers. A single point mutant GIRK4 at position S143 was suggested to form functional homomeric channels (Chan et al., 1996) [31] (Vivaudou et al., 1997) [32]. GIRK channel is predominantly closed at resting membrane potentials. After its activation by the βγ subunit of a G_{i/o} protein, potassium ions flow out of the cell, thus, hyperpolarizing the neuron (Figure 1B). It has therefore been possible to use vertebrate cone opsins SWO (for short wavelength opsin) and LWO (for long wavelength opsin) for repetitive G_{i/o} activation of a specific wavelength *in vivo* in the anxiety circuitry, and the combination of cone opsins with GIRK has proven more efficient that microbial opsins at low light intensities (Masseck et al., 2014) [23].

### Description of the invention

The Inventors have thus investigated if it was possible to implement this cone opsin-based system in a vision restoration setting and investigated patient retinas in preparation for clinical candidate selection. Therefore, in order to develop a light-sensitive cone reactivation strategy, the expression of the phototransduction cascade elements in cones during degeneration was first examined in two RCD mouse models. After exploring the phosphotransduction cascade in two RCD mouse models, a target molecule approach acting via G_{i/o} proteins recruited by the activation of remaining cone opsin was proposed for the first time. It has thereby created a new "short phototransduction cascade" that is independent from the expression of PDE and transducin.

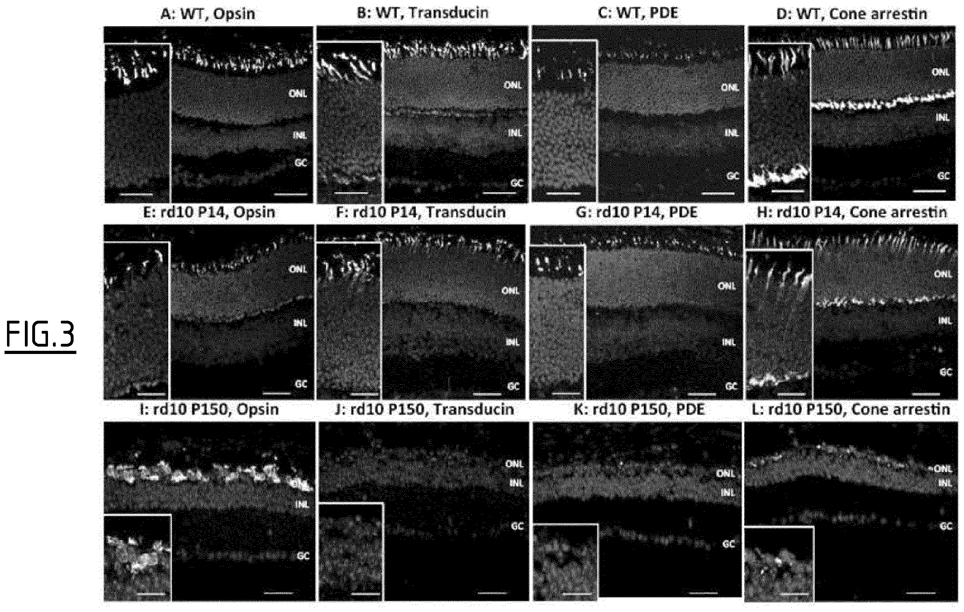

First, the state of the endogeneous phototransduction cascade in degenerating cones through the progression of disease was investigated. In both mouse models, only opsin and arrestin were found to migrate to the cone cell bodies after outer segment degeneration. Thus it was hypothesized that cone reactivation based on cone opsin signaling may be feasible, which in turn will allow to recover high sensitivity vision. It was found that endogenous cone opsins were still expressed at the level of the cone cell body in rd10 mouse model (Figure 3) suggesting the possibility of linking their activity to GIRK channels, such as GIRK2 channel, even in absence of transducin and phosphodiesterase (Figure 1B). In this configuration, the opening of GIRK channel will allow the exit of potassium ions due to the resting membrane potential of dormant cones (Busskamp, 2010) [25]. K⁺ efflux via the GIRK channel will hyperpolarize the cones in response to light as it was seen in the two mouse models of RCD.

Next, GIRK2 channel activated by G proteins recruited by cone opsin was expressed in degenerating cones. Moreover, since the remaining opsin in the cone cell bodies is still functional and sufficient to induce a light response in the degenerated cones, the insertion of GIRK2 in all cones leads to light responses following the spectral properties of each of the opsins preserving color vision. The results pointed towards enhanced light-sensitivity in cones of RCD retinas during and after degeneration of cone outer segments. The results thus demonstrated that vertebrate light sensitive proteins combined with GIRK channel, in particular GIRK2 channel, activated by an endogenous G protein, could improve the visual function in the two mouse models, as demonstrated by electroretinography and behaviour. This is the first time that a light insensitive mammalian ion channel has been linked to intrinsic opsins providing new avenues in vision restoration that can be implemented to increase light sensitivity even before complete outer segment degeneration. Since this new system makes use of intrinsic opsins expressed in degenerating cones, it also enables for the first time, color vision restoration/maintenance.

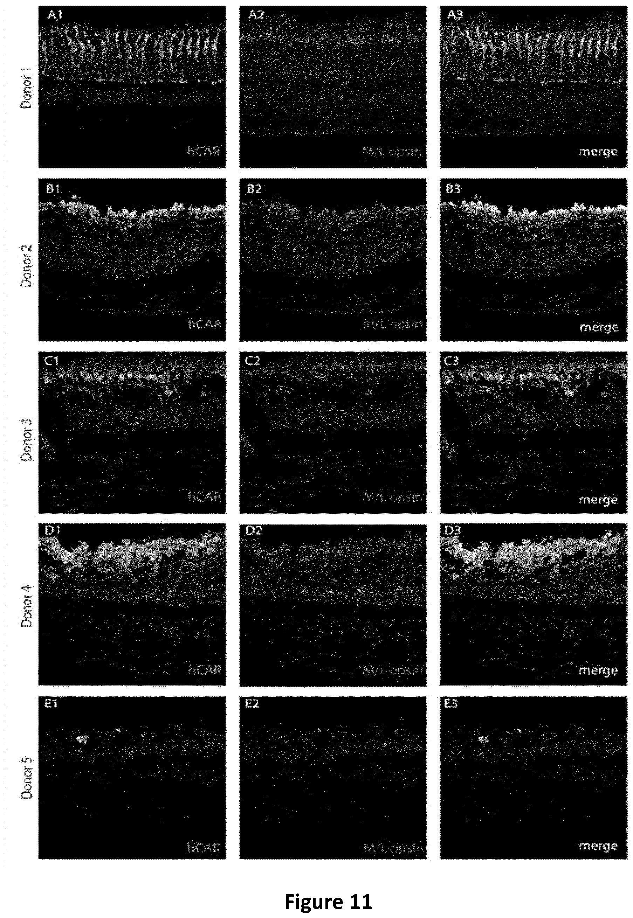

Similarly to the RCD mouse models, the cone opsin and the cone arrestin remain in the cone cell bodies of retinitis pigmentosa (RP) human patients (Figure 11). This result demonstrates the feasibility of reactivating the cone function in the foveal region of RCD human patients with the short GIRK2/opsin phototransduction cascade. The activation of the remaining cone opsin by a light stimulus will trigger the short phototransduction cascade and lead to vision restoration in RCD patients, even at an intermediate or advanced stage of the disease.

This new approach has thus the potential to maintain and/or restore, high acuity and color vision requiring only low light intensities in human patients.

A clear advantage of microbial opsins is their robustness and millisecond scale kinetics (Packer et al., 2013) [26]. For systems using other opsins, it should be considered that in order to respond to another light stimulus, the cascade has to be deactivated to recover light sensitivity. In absence of this, cones may stay hyperpolarized after GIRK2 channel activation limiting their ability to modulate synaptic transmission at a movie rate compatible with motion vision. In the present approach, depolarization of the cones was made possible thanks to the arrestin that is still maintained at very late stages of the disease in both RCD models. This was noticeable in the flicker ERG traces showing responses of the retina during repetitive light stimuli and also by the improved optokinetic reflex of treated mice.

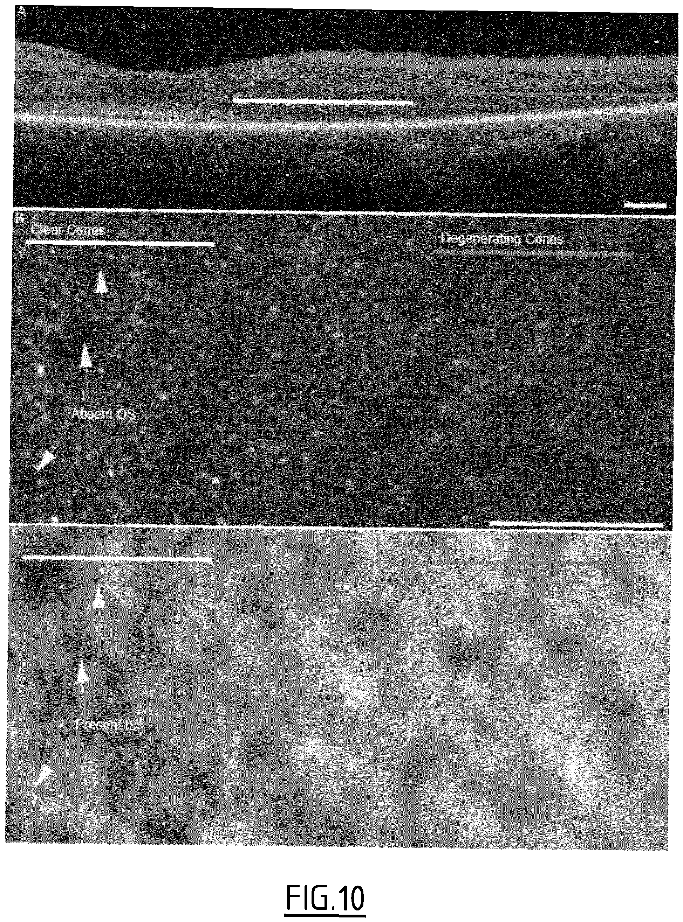

Lastly, the fact that incorporation of GIRK2 enhances existing light responses in cones even prior to complete outer segment loss offers the possibility of implementing this gene therapy in mid stages of the disease. Retinal degeneration in mice is much faster than in humans, thus a few days of therapeutic efficiency in mice is equal to several years in humans. Nonetheless, even when endogenous light responses disappear, retinas expressing GIRK2 are still able to respond to light, generating response amplitudes that correspond to remaining cone numbers. This suggests that patients with remaining foveal cones can benefit from this treatment even if they have no detectable light perception at the beginning of treatment (Figure 10). Thus, this approach can be used as long as cone cells remain. Indeed, a decrease in the response of treated cones to light stimuli was recorded, which was consistent with decrease in cone numbers and the fact that we did not transduce all cones due to subretinal injection further limited the beneficial effect. AAV vectors showing better lateral spread can be used to increase transduced cone numbers beyond the bleb (Khabou et al., 2018; International patent application WO 2018134168) [27, 28]. In order to increase the therapeutic window, neurotrophic factors can be implemented alongside the approach of the present invention. Indeed, AAV-mediated secretion of neurotrophic factors such as the rod-derived cone viability factors (RdCVF), have been shown to delay cone cell death and may be combined with GIRK2 mediated sensitization (Byrne et al., 2015) [29].

It has now been found that a mutated form of GIRK4 channel, GIRK4 S143T, induces significantly more ion efflux than GIRK2 in the context of a short GIRK/opsin phototransduction cascade. This result suggests that incorporation of GIRK4 S143T in cones will provide for an improved gene therapy over GIRK2 gene therapy for the treatment of a retinal degenerative disease such as Rod-cone dystrophies RCD), Cone-rod dystrophies (CRD), Cone dystrophies (CD), and atrophic age-related macular degeneration (AMD).

An object of the present invention is therefore a vector comprising a nucleotide sequence encoding a mutated form of the subunit 4 of G-protein-gated inwardly rectifying potassium channel (GIRK4) (GIRK4 S143T).

The nucleotide sequence encoding GIRK4 S143T can be under the control of a cone-specific promoter such as PR1.7 [33] or a functional variant thereof, or minimal M-opsin promoter, in particular in a pMNTC expression cassette, or a GRK promoter or truncated version thereof (G Protein-Coupled Receptor Kinase, GRK1 in particular).

The vector of the present invention can further comprise a nucleotide sequence encoding a mammalian cone opsin. For example, the mammalian cone opsin is a short wavelength cone opsin (SWO), e.g. from *mus musculus* or human cone opsin. Where present in the same vector, the nucleotide sequence encoding GIRK4 S143T, and the nucleotide sequence encoding a mammalian cone opsin are preferably under the control of a same promoter, in particular a cone-specific promoter such as PR1.7 or a functional variant thereof, or minimal M-opsin promoter, in particular in a pMNTC expression cassette, or a GRK promoter or truncated version thereof (G Protein-Coupled Receptor Kinase, GRK4 in particular).

For the purposes of the present invention, "GIRK4 S143T" means a nucleotide sequence encoding a mutated form of wild-type human subunit 4 of G-protein-gated inwardly rectifying potassium channel (GIRK4) (SEQ ID NO: 1) comprising a substitution of Ser143 by Thr and which retain the ability to respond to light when co-expressed with an opsin. The GIRK4 S143T may differ from wild-type GIRK4 by the substitution of Ser₁₄₃ by Thr, only (e.g. as in SEQ ID NO: 2), or by a limited number of mutation(s), e.g. substitution and/or deletion and/or insertion of at most 1, 2, 3, 4, or 5 of an amino acid(s), in addition to the substitution of Ser₁₄₃ by Thr. For example, a nucleotide sequence encoding GIRK4 S143T comprises or consists of a nucleotide sequence encoding the polypeptide of sequence SEQ ID NO:2, or comprises or consists of the nucleotide sequence SEQ ID NO: 3.

| **Human wild-type GIRK4** | |
|---|---|
| Amino acid sequence (419 AA) | (SEQ ID NO: 1) |
| | |

| | **Human GIRK4 S143T** |
|---|---|
| Nucleotide sequence (1257 NA) | (SEQ ID NO : 3) |
| | ATGGCCGGCGATAGCAGAAACGCCATGAACCAGGACATGGAAATCGGC GTGACCCCTTGGGACCCCAAGAAGATTCCTAAGCAGGCCCGGGACTAC GTGCCAATCGCCACCGATAGAACAAGACTGCTGGCCGAGGGCAAGAAG CCCAGACAGCGGTACATGGAAAAGTCCGGCAAGTGCAACGTGCACCAC GGCAATGTGCAAGAGACATACAGATACCTGAGCGACCTGTTCACCACAC TGGTGGACCTGAAGTGGCGGTTCAACCTGCTGGTGTTTACCATGGTGTA CACAGTGACCTGGCTGTTCTTCGGCTTCATCTGGTGGCTGATCGCCTAC ATCAGAGGCGACCTGGATCACGTGGGCGACCAAGAGTGGATCCCCTGC GTGGAAAATCTGAGCGGCTTCGTGTCCGCCTTCCTGTTCACAATCGAGA CAGAGACAACCATCGGCTACGGCTTCAGAGTGATCACCGAGAAGTGCC CCGAGGGCATCATTCTGCTGCTGGTGCAAGCCATCCTGGGCAGCATCG TGAACGCCTTCATGGTCGGATGCATGTTCGTGAAGATCAGCCAGCCTAA GAAGCGGGCCGAGACACTGATGTTCAGCAACAACGCCGTGATCAGCAT GCGGGACGAGAAGCTGTGCCTGATGTTTAGAGTGGGCGATCTGCGGAA CAGCCACATCGTGGAAGCCAGCATCAGAGCCAAGCTGATCAAGAGCCG GCAGACCAAAGAGGGCGAGTTCATCCCTCTGAACCAGACCGACATCAA CGTGGGCTTCGACACCGGCGACGACAGACTGTTTCTGGTGTCCCCTCT GATCATCTCCCACGAGATCAACCAGAAGTCCCCATTCTGGGAGATGTCT CAGGCCCAGCTGCACCAAGAGGAATTCGAGGTGGTGGTCATCCTGGAA GGCATGGTGGAAGCTACCGGCATGACCTGTCAGGCCAGATCCAGCTAC ATGGACACCGAGGTGCTGTGGGGCCACAGATTCACACCTGTGCTGACC CTGGAAAAGGGCTTCTACGAGGTGGACTACAACACCTTCCACGACACCT ACGAGACAAACACCCCTAGCTGCTGCGCCAAAGAACTGGCCGAGATGA AGAGAGAGGGCAGACTGCTGCAGTACCTGCCTTCTCCACCTCTGCTTG GAGGATGTGCCGAAGCCGGACTGGATGCCGAGGCCGAACAGAACGAG GAAGATGAGCCTAAAGGCCTCGGCGGCAGCAGAGAAGCTAGAGGATCT GTT |
| Amino acid sequence (419 AA) | (SEQ ID NO : 2) |
| | MAGDSRNAMNQDMEIGVTPWDPKKIPKQARDYVPIATDRTRLLAEGKKPR QRYMEKSGKCNVHHGNVQETYRYLSDLFTTLVDLKWRFNLLVFTMVYTVT WLFFGFIWWLIAYIRGDLDHVGDQEWIPCVENLSGFVSAFLFTIETETTIGYG FRVITEKCPEGIILLLVQAILGSIVNAFMVGCMFVKISQPKKRAETLMFSNNAV ISMRDEKLCLMFRVGDLRNSHIVEASIRAKLIKSRQTKEGEFIPLNQTDINVG FDTGDDRLFLVSPLIISHEINQKSPFWEMSQAQLHQEEFEVVVILEGMVEAT GMTCQARSSYMDTEVLWGHRFTPVLTLEKGFYEVDYNTFHDTYETNTPSC CAKELAEMKREGRLLQYLPSPPLLGGCAEAGLDAEAEQNEEDEPKGLGGS REARGSV |

Another object of the present invention is a pharmaceutically acceptable carrier including a vector of the present invention.

According to a particular embodiment of the present invention, the pharmaceutically acceptable carrier can include a vector comprising a nucleotide sequence encoding GIRK4 S143T as described above and a vector comprising a nucleotide sequence encoding a mammalian cone opsin. For example, the mammalian cone opsin is a short wavelength cone opsin (SWO), e.g. from *mus musculus* or human long wavelength-sensitive (OPN1LW), medium wavelength-sensitive (OPN1 MW), short wavelength-sensitive (OPN1SW). According to an embodiment, the mammalian cone opsin is human Long-wave-sensitive opsin 1 (SEQ ID NO:6).

| **Long-wave-sensitive opsin 1 (OPN1LW) homo sapiens** |
|---|
| (SEQ ID NO:6) |
| MVLKAEHTRSPSATLPSNVPSCRSLSSSEDGPSGPSSLADGGLAHNLQDSVRHRILYLSE QLRVEKASRDGNTVSYLKLVSKADRHQVPHIQQAFEKVNQRASATIAQIEHRLHQCHQQL QELEEGCRPEGLLLMAESDPANCEPPSEKALLSEPPEPGGEDGPVNLPHASRPFILESRF QSLQQGTCLETEDVAQQQNLLLQKVKAELEEAKRFHISLQESYHSLKERSLTDLQLLLESL QEEKCRQALMEEQVNGRLQGQLNEIYNLKHNLACSEERMAYLSYERAKEIWEITETFKSR ISKLEMLQQVTQLEAAEHLQSRPPQMLFKFLSPRLSLATVLLVFVSTLCACPSSLISSRLCT CTMLMLIGLGVLAWQRWRAIPATDWQEWVPSRCRLYSKDSGPPADGP |

According to a particular embodiment of the present invention, the pharmaceutically acceptable carrier is for example chosen from solid-lipid nanoparticles, chitosan nanoparticles, liposome, lipoplex or cationic polymer.

According to a particular embodiment of the present invention, the vector of the present invention is a virus, chosen from an adeno-associated virus (AAV), an adenovirus, a lentivirus, an SV40 viral vector. According to a particular embodiment of the present invention, the present invention is equal to or less than 30 nm in size.

Particularly said vector is an adeno-associated virus (AAV).

In the present disclosure, the expression "adeno-associated virus vector" or "AAV vector" has its general meaning in the art.

AAV and AAV vectors have been extensively described in the art as suitable vectors for gene delivery.

Indeed, AAV are non-pathogenic and display a broad range of tissue specificity, depending on their serotype. Typically, AAV according to the present invention are AAV able to target retinal cells. More particularly, AAV according to the present invention are AAV which efficiently transduce retinal cells through intravitreal injection.

For example, it may be an AAV2 or modified versions thereof which efficiently transduce retinal cells through intravitreal injection. A modified version may be for example an AAV comprising an insertion peptide in the capsid protein such as AAV2-7M8 capsid variant as described in the international patent application WO 2012/145601 and in Dalkara *et al.* (2013) [34] which is an AAV2 comprising an insertion peptide called 7m8 in the capsid protein. Other modified versions may be NHP26 [35], NHP9, R100 [36], or other similar variants engineered through directed evolution, rational design and / or machine learning approaches that are commonly known in the art.

Said AAV may also be an AAV serotype or variants which efficiently transduce retinal cells through subretinal injection, such as AAV8 (also referred to as AAV2/8), AAV5 or AAV9-7M8 capsid variant as described in the international patent application WO 2012/145601, which is an AAV9 comprising an insertion peptide called 7m8 in the capsid protein.

As mentioned above, AAVs, such as AAV2, AAV5, AAV8 and AAV9 may be modified by an insertion peptide in the capsid protein. Indeed, they may comprise a variant VP1 capsid protein, wherein the variant AAV capsid protein comprises an insertion peptide of from 7 amino acids to 11 amino acids in the GH loop of said capsid protein relative to a corresponding parental AAV capsid protein.

The insertion peptide may the amino acid sequence LGETTRP (SEQ ID NO: 7) also nicknamed "7m8".

Thus, an AAV2-7m8 capsid variant or AAV9-7m8 capsid variant is an AAV2 or AAV9 comprising a 7 to 11 amino acid long insertion peptide in the GH loop of the VP1 capsid protein, wherein the insertion peptide comprises amino acid sequence LGETTRP (SEQ ID NO: 7) also nicknamed "7m8".

The genomic and polypeptide sequences of various serotypes of AAV, as well as the sequences of the native inverted terminal repeats (ITRs), Rep proteins, and capsid subunits including VP1 protein are known in the art. Such sequences may be found in the literature or in public databases such as GenBank or Protein Data Bank (PDB). See, e.g., GenBank and PDB AF043303 and 1LP3 (AAV2), AY530579 and 3UX1 (AAV9 (isolate hu.14)), the disclosures of which are incorporated by reference herein for teaching AAV nucleic acid and amino acid sequences. Exemplary amino acid sequence of wild-type VP1 for AAV9 and AAV2 are shown in SEQ ID NO: 8 and SEQ ID NO:9, respectively.

| **wild-type AAV9 VP1 capsid protein** |
|---|
| (SEQ ID NO: 8) |
| MAADGYLPDWLEDNLSEGIREWWALKPGAPQPKANQQHQDNARGLVLPGYKYLGPGN GLDKGEPVNAADAAALEHDKAYDQQLKAGDNPYLKYNHADAEFQERLKEDTSFGGNLG RAVFQAKKRLLEPLGLVEEAAKTAPGKKRPVEQSPQEPDSSAGIGKSGAQPAKKRLNFG QTGDTESVPDPQPIGEPPAAPSGVGSLTMASGGGAPVADNNEGADGVGSSSGNWHCD SQWLGDRVITTSTRTWALPTYNNHLYKQISNSTSGGSSNDNAYGYSTPWGYFDFNRFHC HFSPRDWQRLINNNWGFRPKRLNFKLFNIQVKEVTDNNGVKTIANNLTSTVQVFTDSDYQ LPYVLGSAHEGCLPPFPADVFMIPQYGYLTLNDGSQAVGRSSFYCLEYFPSQMLRTGNN FQFSYEFENVPFHSSYAHSQSLDRLMNPLIDQYLYYLSKTINGSGQNQQTLKFSVAGPSN MAVQGRNYIPGPSYRQQRVSTTVTQNNNSEFAWPGASSWALNGRNSLMNPGPAMASH KEGEDRFFPLSGSLIFGKQGTGRDNVDADKVMITNEEEIKTTNPVATESYGQVATNHQSA Q588Q589AQTGWVQNQGILPGMVWQDRDVYLQGPIWAKIPHTDGNFHPSPLMGGFGMKH PPPQILIKNTPVPADPPTAFNKDKLNSFITQYSTGQVSVEIEWELQKENSKRWNPEIQYTS NYYKSNNVEFAVNTEGVYSEPRPIGTRYLTR |

| **wild-type AAV2 VP1 capsid protein** |
|---|
| (SEQ ID NO: 9) |
| |

Preferably, the insertion site of the insertion peptide in the GH loop of the VP1 capsid protein is between amino acids 587 and 588 of AAV2 wild-type VP1 capsid protein, between amino acids 588 and 589 of AAV9 wild-type VP1 capsid protein.

According to some embodiments, the insertion peptide has a length of 7 amino acids, 8 amino acids, 9 amino acids, 10 amino acids, or 11 amino acids.

The insertion peptide may comprise one or more spacer amino acids at the N- and/or C-terminus of amino acid sequence LGETTRP (SEQ ID NO: 7). Preferably, the spacer amino acids are selected from the group consisting of Ala, Leu, Gly, Ser, and Thr, more preferably from the group consisting of Ala, Leu, and Gly.

According to an embodiment, the insertion peptide comprises or consists of sequence AALGETTRPA (SEQ ID NO: 10), LALGETTRPA (SEQ ID NO: 11), or GLGETTRPA (SEQ ID NO: 12), preferably comprises or consists of sequence AALGETTRPA (SEQ ID NO: 10) or LALGETTRPA (SEQ ID NO: 11). Indeed, all these insertion peptides comprise the amino acid sequence LGETTRP (SEQ ID NO: 7).

The insertion peptide may also be one of the following sequences: NETITRP (SEQ ID NO: 14), KAGQANN (SEQ ID NO: 15), KDPKTTN (SEQ ID NO: 16), KDTDTTR (SEQ ID NO: 17), RAGGSVG (SEQ ID NO: 18), AVDTTKF (SEQ ID NO: 19), STGKVPN (SEQ ID NO: 20), LAISDQTKHA (SEQ ID NO:21).

In one embodiment, the AAV and the AAV vector according to the present invention is obtained according to the method described in international patent application WO2012/158757.

In a particular embodiment, the AAV capsid is obtained according to the method described in patent application US9193956B2.

According to a particular embodiment, the viral vector, in particular an AAV such as an AAV2 or modified versions thereof which efficiently transduce retinal cells through intravitreal injection, such as AAV2-7M8, NHP26, NHP9, or R100, or a AAV serotype or variant which efficiently transduce retinal cells through subretinal injection, such as AAV8, AAV5 or AAV9-7M8, comprises the polynucleotide of interest (nucleotide sequence encoding GIRK4 S143T or a functional derivative thereof, and/or nucleotide sequence encoding mammalian cone opsin) under the control of a cone-specific promoter, preferably a pR1.7 or a functional variant thereof, or a minimal M-opsin promoter, in particular in a pMNTC expression cassette. In said AAV, the polynucleotide of interest which is operatively linked to the cone-specific promoter, e.g. promoter pR1.7, minimal M-opsin promoter or pMNTC, is preferably flanked by two adeno-associated virus inverted terminal repeats (AAV ITRs), preferably AAV2 ITRs.

PR1.7 is a 1.7 kilobases synthetic promoter based on the human red opsin promoter sequence described in Ye et al. [33]. As used herein, "PR1.7" denotes the promoter of sequence SEQ ID NO:13 and functional variants thereof. "Functional variants" of the PR1.7 promoter typically have one or more nucleotide mutations (such as a nucleotide deletion, addition, and/or substitution) relative to the native PR1.7 promoter (SEQ ID NO: 13), which do not significantly alter the transcription of the polynucleotide of interest. In the context of the present invention, said functional variants retain the capacity to drive a strong expression, in cone photoreceptors, of the polynucleotide of interest. Such capacity can be tested as described by Ye et al. [33] and Khabou et al. [27].

Another example of cone-specific promoter which may be used is a minimal M-opsin promoter region such as disclosed in WO2015142941, in particular in SEQ ID NO:55 or SEQ ID NO: 93 as disclosed in WO2015142941. Instant sequence SEQ ID NO:4 is identical to SEQ ID NO: 93 of WO2015142941.

In an embodiment, the polynucleotide of interest which is placed under the control the minimal M-opsin promoter region, is inserted in a pMNTC expression cassette (SEQ ID NO:5) comprising an optimized enhancer, optimized promoter, optimized 5'UTR, optimized intron, optimized kozak and optimized polyA region (SEQ ID NO:95 of WO2015142941).

| **PR1.7 promoter** |
|---|
| (SEQ ID NO:13) |
| ggaggctgaggggtggggaaagggcatgggtgtttcatgaggacagagcttccgtttcatgcaatgaaaagagtttggagac ggatggtggtgactggactatacacttacacacggtagcgatggtacactttgtattatgtatattttaccacgatctttttaaagtgt caaaggcaaatggccaaatggttccttgtcctatagctgtagcagccatcggctgttagtgacaaagcccctgagtcaagatg acagcagcccccataactcctaatcggctctcccgcgtggagtcatttaggagtagtcgcattagagacaagtccaacatcta atcttccaccctggccagggccccagctggcagcgagggtgggagactccgggcagagcagagggcgctgacattgggg cccggcctggcttgggtccctctggcctttccccaggggccctctttccttggggctttcttgggccgccactgctcccgctcctctc cccccatcccaccccctcaccccctcgttcttcatatccttctctagtgctccctccactttcatccacccttctgcaagagtgtggg accacaaatgagttttcacctggcctggggacacacgtgcccccacaggtgctgagtgactttctaggacagtaatctgctttag gctaaaatgggacttgatcttctgttagccctaatcatcaattagcagagccggtgaaggtgcagaacctaccgcctttccaggc ctcctcccacctctgccacctccactctccttcctgggatgtgggggctggcacacgtgtggcccagggcattggtgggattgca ctgagctgggtcattagcgtaatcctggacaagggcagacagggcgagcggagggccagctccggggctcaggcaaggc tgggggcttcccccagacaccccactcctcctctgctggacccccacttcatagggcacttcgtgttctcaaagggcttccaaat agcatggtggccttggatgcccagggaagcctcagagttgcttatctccctctagacagaaggggaatctcggtcaagaggg agaggtcgccctgttcaaggccacccagccagctcatggcggtaatgggacaaggctggccagccatcccaccctcagaa gggacccggtggggcaggtgatctcagaggaggctcacttctgggtctcacattcttggatccggttccaggcctcggccctaa atagtctccctgggctttcaagagaaccacatgagaaaggaggattcgggctctgagcagtttcaccacccaccccccagtct gcaaatcctgacccgtgggtccacctgccccaaaggcggacgcaggacagtagaagggaacagagaacacataaacac agagagggccacagcggctcccacagtcaccgccaccttcctggcggggatgggtggggcgtctgagtttggttcccagca aatccctctgagccgcccttgcgggctcgcctcaggagcaggggagcaagaggtgggaggaggaggtctaagtcccaggc ccaattaagagatcaggtagtgtagggtttgggagcttttaaggtgaagaggcccgggctgatcccacaggccagtataaag cgccgtgaccctcaggtgatgcgccagggccggctgccgtcggggacagggctttccatagcc |

| **minimal M-opsin promoter region** |
|---|
| (SEQ ID NO :4) |
| Ccagcaaatccctctgagccgcccccgggggctcgcctcaggagcaaggaagcaaggggtgggaggaggaggtctaag tcccaggcccaattaagagatcagatggtgtaggatttgggagcttttaaggtgaagaggcccgggctgatcccactggccgg tataaagcaccgtgaccctcaggtgacgcaccagggccggctgccgtcggggacagggctttccatagcccag |

| **pMNTC** |
|---|
| (SEQ ID NO :5) |
| cctacagcagccagggtgagattatgaggctgagctgagaatatcaagactgtaccgagtagggggccttggcaagtgtgg agagcccggcagctggggcagagggcggagtacggtgtgcgtttacggacctcttcaaacgaggtaggaaggtcagaagt caaaaagggaacaaatgatgtttaaccacacaaaaatgaaaatccaatggttggatatccattccaaatacacaaaggcaa cggataagtgatccgggccaggcacagaaggccatgcacccgtaggattgcactcagagctcccaaatgcataggaatag aagggtgggtgcaggaggctgaggggtggggaaagggcatgggtgtttcatgaggacagagcttccgtttcatgcaatgaa aagagtttggagacggatggtggtgactggactatacacttacacacggtagcgatggtacactttgtattatgtatattttaccac gatctttttaaagtgtcaaaggcaaatggccaaatggttccttgtcctatagctgtagcagccatcggctgttagtgacaaagccc ctgagtcaagatgacagcagcccccataactcctaatcggctctcccgcgtggagtcatttaggagtagtcgcattagagaca agtccaacatctaatcttccaccctggccagggccccagctggcagcgagggtgggagactccgggcagagcagagggc gctgacattggggcccggcctggcttgggtccctctggcctttccccaggggccctctttccttggggctttcttgggccgccactg ctcccgctcctctccccccatcccaccccctcaccccctcgttcttcatatccttctctagtgctccctccactttcatccacccttctg caagagtgtgggaccacaaatgagttttcacctggcctggggacacacgtgcccccacaggtgctgagtgactttctaggaca gtaatctgctttaggctaaaatgggacttgatcttctgttagccctaatcatcaattagcagagccggtgaaggtgcagaacctac cgcctttccaggcctcctcccacctctgccacctccactctccttcctgggatgtgggggctggcacacgtgtggcccagggcat tggtgggattgcactgagctgggtcattagcgtaatcctggacaagggcagacagggcgagcggagggccagctccgggg ctcaggcaaggctgggggcttcccccagacaccccactcctcctctgctggacccccacttcatagggcacttcgtgttctcaa agggcttccaaatagcatggtggccttggatgcccagggaagcctcagagttgcttatctccctctagacagaaggggaatct cggtcaagagggagaggtcgccctgttcaaggccacccagccagctcatggcggtaatgggacaaggctggccagccatc ccaccctcagaagggacccggtggggcaggtgatctcagaggaggctcacttctgggtctcacattcttccagcaaatccctct gagccgcccccgggggctcgcctcaggagcaaggaagcaaggggtgggaggaggaggtctaagtcccaggcccaatta agagatcagatggtgtaggatttgggagcttttaaggtgaagaggcccgggctgatcccactggccggtataaagcaccgtg accctcaggtgacgcaccagggccggctgccgtcggggacagggctttccatagcccaggtaagtatcaaggttacaagac aggtttaaggagaccaatagaaactgggcttgtcgagacagagaagactcttgcgtttctgataggcacctattggtcttactga catccactttgcctttctctccacaggcccagagaggagacaggccgccacc |

The promoter and the polynucleotide of interest are operatively linked. As used herein, the term "operably linked" refers to two or more nucleic acid or amino acid sequence elements that are physically linked in such a way that they are in a functional relationship with each other. For instance, a promoter is operably linked to a coding sequence if the promoter is able to initiate or otherwise control/regulate the transcription and/or expression of a coding sequence, in which case the coding sequence should be understood as being "under the control of" the promoter. Generally, when two nucleic acid sequences are operably linked, they will be in the same orientation and usually also in the same reading frame. They will usually also be essentially contiguous, although this may not be required.

According to an embodiment, the viral vector is an AAV, such as an AAV2 or modified versions thereof which efficiently transduce retinal cells through intravitreal injection, such as AAV2-7M8, NHP26, NHP9, or R100. The viral vector may also be an AAV serotype or variant which efficiently transduce retinal cells through subretinal injection, such as AAV8 or AAV9-7M8.

In particular, the AAV is an AAV9 (AAV9-7m8-PR1.7) or an AAV2 (AAV2-7m8-PR1.7) comprising:
- a VP1 capsid protein in which a 7 to 11 amino acid long insertion peptide is inserted in the GH loop of said VP1 capsid protein relative to wild-type AAV9 VP1 capsid protein, at a position localized between amino acids 588 and 589 of wild-type AAV9 VP1 capsid protein, wherein said peptide comprises amino acid sequence LGETTRP (SEQ ID NO: 7); and
- the polynucleotide of interest (nucleotide sequence encoding GIRK4 S143T and/or nucleotide sequence encoding mammalian cone opsin) under the control of a PR1.7 promoter.

In said AAV9-7m8 or AAV2-7m8, the insertion peptide has a length of 7 amino acids, 8 amino acids, 9 amino acids, 10 amino acids, or 11 amino acids. Preferably, the insertion peptide comprises one or more spacer amino acids at the N- and/or C-terminus of amino acid sequence LGETTRP (SEQ ID NO: 7). Preferably, the spacer amino acids are selected from the group consisting of Ala, Leu, Gly, Ser, and Thr, more preferably from the group consisting of Ala, Leu, and Gly. According to an embodiment, the insertion peptide comprises or consists of sequence AALGETTRPA (SEQ ID NO: 10), LALGETTRPA (SEQ ID NO: 11), or GLGETTRPA (SEQ ID NO: 12); preferably comprises or consists of sequence AALGETTRPA (SEQ ID NO: 10) or LALGETTRPA (SEQ ID NO: 11).

The vectors of the invention are produced using methods known in the art. In short, the methods generally involve (a) the introduction of the AAV vector into a host cell, (b) the introduction of an AAV helper construct into the host cell, wherein the helper construct comprises the viral functions missing from the AAV vector and (c) introducing a helper virus into the host cell. All functions for AAV virion replication and packaging need to be present, to achieve replication and packaging of the AAV vector into AAV virions. The introduction into the host cell can be carried out using standard virology techniques simultaneously or sequentially. Finally, the host cells are cultured to produce AAV virions and are purified using standard techniques such as iodixanol or CsCI gradients or other purification methods. The purified AAV virion is then ready for use.

Another object of the present invention is a nucleic acid comprising a nucleotide sequence encoding GIRK4 S143T as described above, for use as a medicament. In particular, said nucleic acid is for use in treating retinal degenerative diseases.

In the context of the invention, the term "treating" or "treatment", as used herein, means reversing, alleviating, inhibiting the progress of, or preventing the disorder or condition to which such term applies, or one or more symptoms of such disorder or condition (e.g., retinal degenerative diseases).

In the present application, the term "retinal degenerative disease", also called "[8] Retinal neurodegenerative disorders" encompasses all diseases associated with rods and cones degeneration. It encompasses different subgroups of pathologies: Rod-cone dystrophies (RCD), Cone-rod dystrophies (CRD), Cone dystrophies (CD), and atrophic age-related macular degeneration (AMD).

Rod-cone dystrophies (RCD), such as retinitis pigmentosa (RP), are genetically heterogeneous retinal neurodegenerative diseases characterized by the progressive death of rod photoreceptors followed by the consecutive loss of cones. RP is one of the most common forms of inherited retinal degeneration, affecting around 1:3,500 people worldwide, which represents 2 million patients worldwide. Mutations causing RP in over 63 distinct genes have been identified to date with a significant proportion of these mutations in rod-specific transcripts.

Cones dystrophies are characterized by the vision loss (age of onset ranging from the late teens to the sixties), sensitivity to bright lights, and poor color vision. Therefore, patients see better at dusk. Visual acuity usually deteriorates gradually, but it can deteriorate rapidly to 20/200. Later, in more severe cases, it drops to "counting fingers" vision. Color vision testing using color test plates (HRR series) reveals many errors on both red-green and blue-yellow plates.

Cone-Rod Dystrophies (CRD) refer to a group of inherited retinal degenerations (1:30 - 40,000 people) that affect the photoreceptor (light sensing) cells that are responsible for capturing images from the visual field. These cells line the back of the eye in the region known as the retina. Cone photoreceptor cells are present throughout the retina but are concentrated in the central region (the macula). They are useful for central (reading) vision. Rod photoreceptor cells are present throughout the retina except for the very center of the macula called the fovea where only cones are present. They are responsible for night vision.

In contrast to typical retinitis pigmentosa (known as the Rod-Cone Dystrophies), which results from the loss of rod cells and later the cone cells, Cone-Rod Dystrophies can reflect the opposite sequence of events, where cone cells are primarily first affected with later loss of rods. The degree of vision loss becomes more severe over time. There are multiple types of Cone-Rod Dystrophies, which are determined by their genetic cause and pattern of inheritance.

Retinal degenerative diseases include but are not limited to retinitis pigmentosa, age-related macular degeneration, Bardet-Biedel syndrome, Bassen-Kornzweig syndrome, Best disease, choroideremia, gyrate atrophy, Leber congenital amaurosis, Refsum disease, Stargardt disease or Usher syndrome.

In a particular embodiment, the retinal degenerative disease is a rod-cone dystrophy, more particularly retinitis pigmentosa, in particular non-syndromic X-linked Retinitis Pigmentosa (XLRP), autosomal recessive RP or autosomal dominant RP.

In a particular embodiment, the retinal degenerative disease is a cone-rod dystrophy, more particularly Stargardt disease, X Linked cone dystrophy, and Bardet-Biedl syndrome.

In other words, another object of the present invention is a method of treating retinal degenerative disease such as Rod-cone dystrophies RCD), Cone-rod dystrophies (CRD), Cone dystrophies (CD), and atrophic age-related macular degeneration (AMD) in a mammal in need thereof, the method comprising administering to the mammal a nucleic acid comprising a nucleotide sequence encoding GIRK4 S143T as described above.

In an embodiment, the nucleic acid comprising a nucleotide sequence encoding GIRK4 S143T comprises or consists of a nucleotide sequence encoding the polypeptide of sequence SEQ ID NO: 2, or comprises or consists of the nucleotide sequence SEQ ID NO: 3.

The nucleic acid comprising a nucleotide sequence encoding GIRK4 S143T may be in a vector selected from the group consisting of an adeno-associated virus (AAV), an adenovirus, a lentivirus, and SV40 viral vector.

According to an embodiment, the nucleic acid comprising a nucleotide sequence encoding GIRK4 S143T is under the control of PR1.7 promoter or of a functional variant of said promoter.

According to an embodiment, the nucleic acid comprising a nucleotide sequence encoding GIRK4 S143T is in a vector which is an AAV. In particular, said AAV may be an AAV2 or modified versions thereof which efficiently transduce retinal cells through intravitreal injection, such as AAV2-7M8 capsid variant as described in the international patent application WO 2012/145601 and in Dalkara *et al.* (2013) [34], NHP26 [35], NHP9, R100 [36], or other similar variants engineered through directed evolution, rational design and / or machine learning approaches that are commonly known in the art. In particular, said AAV may also be an AAV serotype or variants which efficiently transduce retinal cells through subretinal injection, such as AAV8 or AAV9-7M8 capsid variant as described in the international patent application WO 2012/145601.

In particular, the AAV is an AAV2 or AAV9 virus comprising a 7 to 11 amino acid long insertion peptide in the GH loop of the VP1 capsid protein, wherein the insertion peptide comprises amino acid sequence LGETTRP (SEQ ID NO: 7). In other words, the AAV is an AAV2-7M8 or an AAV9-7M8 according to the definitions previously given.

More particularly, the vector is a recombinant AAV2 or AAV9 vector comprising:
- a VP1 capsid protein in a 7 to 11 amino acid long insertion peptide is inserted in the GH loop of said VP1 capsid protein relative to wild-type AAV9 VP1 capsid protein, at a position localized between amino acids 588 and 589 of wild-type AAV9 VP1 capsid protein, wherein said peptide comprises amino acid sequence LGETTRP (SEQ ID NO: 7); and
- the nucleotide sequence encoding the mammalian cone opsin under the control of a PR1.7 promoter.

According to an embodiment, said insertion peptide comprises or consists of amino acid sequence AALGETTRPA (SEQ ID NO: 8), LALGETTRPA (SEQ ID NO: 9), or GLGETTRPA (SEQ ID NO: 10).

According to an embodiment, the nucleic acid comprising a nucleotide sequence encoding GIRK4 S143T further comprises a sequence encoding a mammalian cone opsin, as described above.

According to another embodiment, the nucleic acid comprising a nucleotide sequence encoding GIRK4 S143T is for use in treating retinal degenerative disease such as Rod-cone dystrophies RCD), Cone-rod dystrophies (CRD), Cone dystrophies (CD), and atrophic age-related macular degeneration (AMD) in combination with another nucleic acid encoding a mammalian cone opsin, as described above. In other words, another object of the present invention is a method of treating retinal degenerative disease such as Rod-cone dystrophies RCD), Cone-rod dystrophies (CRD), Cone dystrophies (CD), and atrophic age-related macular degeneration (AMD) in a mammal in need thereof, the method comprising administering to the mammal a nucleic acid comprising a nucleotide sequence encoding GIRK4 S143T and another nucleic acid encoding a mammalian cone opsin. In said method, the nucleic acids are therefore in separate forms and may be formulated in a single pharmaceutical composition or in two separate pharmaceutical compositions which may be administered simultaneously of separately over time.

Another object of the present invention is a pharmaceutical composition comprising the vector as previously described or a pharmaceutically acceptable carrier including said vector, with a pharmaceutically acceptable carrier, diluent or excipient.

Another object of the present invention is a vector, a carrier or a pharmaceutical composition of the present invention, for use in treating retinal degenerative disease such as Rod-cone dystrophies RCD), Cone-rod dystrophies (CRD), Cone dystrophies (CD), and atrophic age-related macular degeneration (AMD).

Retinal degenerative diseases include but are not limited to retinitis pigmentosa, age-related macular degeneration, Bardet-Biedel syndrome, Bassen-Kornzweig syndrome, Best disease, choroideremia, gyrate atrophy, Leber congenital amaurosis, Refsum disease, Stargardt disease or Usher syndrome.

In a particular embodiment, the retinal degenerative disease is a rod-cone dystrophy, more particularly retinitis pigmentosa, in particular non-syndromic X-linked Retinitis Pigmentosa (XLRP), autosomal recessive RP or autosomal dominant RP.

In a particular embodiment, the retinal degenerative disease is a cone-rod dystrophy, more particularly Stargardt disease, X Linked cone dystrophy, and Bardet-Biedl syndrome.
diseases such as Retinitis Pigmentosa (RP), in particular non-syndromic X-linked Retinitis Pigmentosa (XLRP), autosomal recessive RP, autosomal dominant RP. The subject suffering from a retinal degenerative disease to be treated is a mammal, in particular a non-human or human primate, preferably a human. The disease in the mammal may be at an early, intermediate or advanced stage of the disease. In subjects suffering from a retinal degenerative disease at intermediate or advanced stage of the disease, transduction of the subjects' cones with a nucleotide sequence encoding GIRK4 S143T would be sufficient to achieve vision restoration provided cone opsin and cone arrestin are still expressed in the patients' cone cell bodies. In subjects suffering from a retinal degenerative disease whose cone cell bodies no longer express cone opsin, transduction of the subjects' cones with a nucleotide sequence encoding GIRK4 S143T and a mammalian cone opsin would be required.

Accordingly, treatment of retinal degenerative diseases may be implemented by administering the vector(s), carrier or pharmaceutical composition of the present invention to the mammal, so as to achieve transduction of cones with the GIRK4 S143T transgene, or GIRK4 S143T and mammalian cone opsin transgenes.

In other words, another object of the present invention is a method of treating a retinal degenerative disease such as RCD, CRD, CD or AMD, in a mammal in need thereof, the method comprising administering to the mammal an effective amount of the vector or the carrier of the pharmaceutical composition of the present invention.

Accordingly, in a first embodiment, the vector comprising a nucleotide sequence encoding GIRK4 S143T, carrier including said vector, or a pharmaceutical composition comprising the vector or carrier is for use in treating a retinal degenerative disease such as RCD, CRD, CD or AMD, in a mammalian subject whose cone cells still express endogenous cone opsin. According to an embodiment, the vector further comprises a nucleotide sequence encoding a mammalian cone opsin. According to another embodiment, the vector does not comprise a nucleotide sequence encoding a mammalian cone opsin. According to an embodiment, the carrier further includes a vector comprising a nucleotide sequence encoding a mammalian cone opsin. According to another embodiment, the carrier does not include a vector comprising a nucleotide sequence encoding a mammalian cone opsin.

In a second embodiment, the vector comprising a nucleotide sequence encoding GIRK4 S143T, carrier including said vector, or a pharmaceutical composition comprising the vector or carrier is for use in treating a retinal degenerative disease such as RCD, CRD, CD or AMD, in a mammalian subject whose cone cells no longer express endogenous cone opsin. According to this embodiment, the vector further comprises a nucleotide sequence encoding a mammalian cone opsin, or the carrier further includes a vector comprising a nucleotide sequence encoding a mammalian cone opsin.

Treatment of retinal degenerative diseases may also be implemented by transducing a mammalian cone precursor cell with vector(s), carrier or pharmaceutical composition of the present invention, and administering the transduced mammalian cone precursor cell to the retina, in particular to the fovea region, of the mammal to be treated.

In other words, another object of the present invention is a method of treating a retinal degenerative disease such as RCD, CRD, CD or AMD in a mammal in need thereof, the method comprising administering to the mammal an effective amount mammalian cone precursor cell transduced with the vector or the carrier of the pharmaceutical composition of the present invention.

The invention also relates to a cone precursor cell comprising a heterologous nucleic acid encoding GIRK4 S143T, or encoding GIRK4 S143T and a mammalian cone opsin, for use in a method of treating a retinal degenerative disease such as RCD, CRD, CD or AMD. Accordingly, it is also provided a method of treating a a mammal in need thereof, the method comprising administering to the mammal a cone precursor cell comprising a heterologous nucleic acid encoding GIRK4 S143T, or encoding GIRK4 S143T and a mammalian cone opsin. As used herein, the term « heterologous nucleic acid » refers to a gene, polynucleotide or nucleic acid sequence that is not in its natural environment.

Cone precursor cells are not-fully differentiated, non-dividing cells committed to differentiate into cone cells.

In an embodiment, cone precursor cells are obtained from retina of donor (e.g. cadaver eye donor) or from the subject suffering from a retinal degenerative disease to be treated, preferably from the subject suffering from a retinal degenerative disease to be treated. In another embodiment, cone precursor cells are obtained from stem cells, in particular embryonic stem cells, induced pluripotent stem (iPS cells), adult stem cells or fetal stem cells. In another embodiment, cone precursor cells are obtained from differentiated embryonic stem cells. According to one embodiment, embryonic stem cells are non-human embryonic stem cells. According to another embodiment, human embryonic stem cells may be used with the proviso that the method itself or any related acts do not include destruction of human embryos. Preferably cone precursor cells are obtained by differentiation of stem cells, preferably from differentiation of adult stem cells or induced pluripotent stem cells, more preferably from differentiation of induced pluripotent stem cells obtained from somatic cells, e.g. fibroblasts, of the subject suffering from a retinal degenerative disease to be treated.

Embryonic stem cells are able to maintain an undifferentiated state or can be directed to mature along lineages deriving from all three germ layers, ectoderm, endoderm and mesoderm. Embryonic stem cells can be reprogrammed towards cone photoreceptors by manipulation of key developmental signaling pathways as described in the international patent application WO2018055131. For example, it may be used antagonists of the nodal and wnt pathway in addition to activin-A and serum (Watanabe K et al. Nat Neurosci. 2005 Mar;8(3):288-96.), or inhibition of the Notch signaling pathway can be implemented (Osakada F et al. Nat Protoc. 200;4(6):811-24). Cone precursor cells can be obtained from embryonic stem cells using any protocol known by the skilled person (Osakada F et al. Nat Biotechnol. 2008 Feb;26(2):215-24; Amirpour N et al. Stem Cells Dev. 2012 Jan;21(I):42-53; Nakano T et al. Cell Stem Cell. 2012 Jun 14;10(6):771-85; Zhu Y et al. Plos One. 25 2013;8(l):e54552; Yanai A et al. Tissue Eng Part C Methods. 2013 Oct;19(10):755-64; Kuwahara A et al. Nat Commun. 2015 Feb 19;6:6286; Mellough CB et al. Stem Cells. 2015 Aug;33(8):2416-30; Singh K et al. Stem Cells Dev. 2015 Dec I;24(23):2778-95).

Preferably, cone precursor cells are obtained from iPS cells or adult stem cells, more preferably from iPS cells. Induced pluripotent stem (iPS) cells are derived from a non-pluripotent cell, typically an adult somatic cell, by a process known as reprogramming, where the introduction of only a few specific genes are necessary to render the cells pluripotent (e.g. OCT4, SOX2, KLF4 and C-MYC in human cells). One benefit of use of iPS cells is the avoidance of the use of embryonic cells altogether and hence any ethical questions thereof. Photoreceptor precursor cells can be obtained from iPS cells using any differentiation method known by the skilled person.

In particular, photoreceptor precursor cells can be obtained from human iPS cells by a method as disclosed in Garita-Hernandez et al., 2019, Nat Commun 10, 4524. Human iPS are expanded to confluence in iPS medium (e.g. Essential 8^{™} medium, GIBCO, Life Technologies). After 80% confluence, the medium was switched to a proneural medium (e.g. Essential 6^{™} medium supplemented with 1 % N2 supplement (100X); GIBCO, Life Technologies). The medium was changed every 2-3 days. After 4 weeks of differentiation, neural retina-like structures grew out of the cultures and were mechanically isolated. Pigmented parts, giving rise to RPE were carefully removed. The extended 3D culture in Maturation medium (DMEM/F-12 medium supplemented with 2% B-27^{™} Supplement (50X), serum free, and 1% MEM Non-Essential Amino Acids Solution (100X) ; GIBCO, Life Technologies) allowed the formation of retinal organoids. Addition of 10 ng/ml Fibroblast growth factor 2 (FGF2, Preprotech) at this point favoured the growth of retinal organoids and the commitment towards retinal neurons instead of RPE lineage. In order to promote the commitment of retinal progenitors towards photoreceptors, Notch signalling was specifically blocked for a week starting at day 42 of differentiation using the gamma secretase inhibitor DAPT (10 µM, Selleckchem). Floating organoids were cultured in 6 well-plates (10 organoids per well) and medium was changed every 2 days.

Photoreceptor precursor cells can also be obtained from human iPS cells using any other protocol known by the skilled person (Lamba, Osakada and colleagues (Lamba et al. Proc Natl Acad Sci USA. 2006 Aug 22;103(34):12769-74;, Lamba et al. Plos one. 2010 Jan 20;5(I):e8763; Osakada et al. Nat. Protoc. 2009;4(6):811-24; Meyer JS et al. Proc Natl Acad Sci USA. 2009 Sep 29; 106(39):16698-703 ; Meyer JS et al. Stem Cells. 2011 Aug ;29(8) :1206-18; Mellough CB et al. Stem Cells. 2012 Apr;30(4):673-86; Boucherie C et al. Stem Cells. 2013 Feb ;31(2) :408-14 ; Sridhar A et al. Stem Cells Transl Med. 2013 ;2(4) :255-64 ; Tucker BA et al. Elife. 2013 Aug 27,2:e00824 ; Tucker BA et al. Stem Cells Transl Med. 2013 Jan ;2(1) :16-24 ; eichman S et al. Proc Natl Acad Sci USA. 2014 Jun 10 ;111(23) :8518-23 ; Zhong X et al. Nat Commin. 2014 Jun 10 ;5 :4047 ; Wang X et al. Biomaterials. 2015 Jun ;53 :40-9).

The cone precursor comprises a heterologous nucleic acid encoding i) GIRK4 S143T, or ii) encoding GIRK4 S143T and a mammalian cone opsin. Where the cone precursor cells comprise a heterologous nucleic acid encoding GIRK4 S143T, or a functional derivative thereof, and a mammalian cone opsin, the cone precursor cells either comprise i) a heterologous nucleic acid encoding both GIRK4 S143T and a mammalian cone opsin, or ii) a heterologous nucleic acid encoding GIRK4, and another heterologous nucleic acid encoding a mammalian cone opsin.

Said cone precursor cells may be prepared by introducing into said cone precursor cells said heterologous nucleic acid(s), or an expression cassette or vector comprising said nucleic acid(s), by any method known to the skilled person. According to an embodiment, a cone precursor cell comprising a heterologous nucleic acid encoding GIRK4 S143T, or encoding GIRK4 S143T and a mammalian cone opsin, is prepared by infecting the cone precursor cell with a viral vector as described above. Particularly said vector is an adeno-associated virus (AAV). Said AAV may be an AAV2 or modified versions thereof which efficiently transduce retinal cells through intravitreal injection, such as AAV2-7M8 capsid variant as described in the international patent application WO 2012/145601 and in Dalkara *et al.* (2013) [34], NHP26 [35], NHP9, R100 [36], or other similar variants engineered through directed evolution, rational design and / or machine learning approaches that are commonly known in the art. Said AAV may also be an AAV serotype or variants which efficiently transduce retinal cells through subretinal injection, such as AAV8 or AAV9-7M8 capsid variant as described in the international patent application WO 2012/145601.

In another aspect, the invention therefore further refers to a method of preparing a cone precursor cell comprising a heterologous nucleic acid encoding GIRK4 S143T, or encoding GIRK4 S143T and a mammalian cone opsin, said method comprising infecting cone precursor cells with a viral vector or carrier according to the invention, and recovering infected cone precursor cells.

The vector, carrier, or pharmaceutical composition, or cone precursor cells may be administered by any suitable route known to the skilled person in particular by intravitreal or subretinal or suprachoroidal administration.

In an embodiment, the viral vector, carrier or pharmaceutical composition is injected at a dose comprised between 10⁷ to 10¹⁵ vg/eye, preferably between 10¹¹ and 10¹⁵ vg/eye, even more preferably 10¹¹ and 10¹³ vg/eye.

In an embodiment, the viral vector, carrier or pharmaceutical composition is administered by intravitreal injection.

In particular, the AAV vector is an AAV2 or modified versions thereof which efficiently transduce retinal cells through intravitreal injection, such as AAV2-7M8 capsid variant as described in the international patent application WO 2012/145601 and in Dalkara *et al.* (2013) [34], NHP26 [35], NHP9, R100 [36], or other similar variants engineered through directed evolution, rational design and / or machine learning approaches that are commonly known in the art, and said vector is administered by intravitreal injection.

In an embodiment, the viral vector, carrier or pharmaceutical composition is administered by subretinal injection.

In particular, the AAV vector is an AAV serotype or variants which efficiently transduce retinal cells through subretinal injection, such as AAV8 or AAV9-7M8 capsid variant as described in the international patent application WO 2012/145601.

The fovea is a small region in the central retina of primates of approximately equal to or less than 0.5 mm in diameter that contains only cone photoreceptor cells, and highest density of cones in the whole retina. The fovea dominates the visual perception of primates by providing high-acuity color vision. The highest density of cones is found at the center of the fovea (<0.3 mm from the foveal center), devoid of rod photoreceptors. Cone density decreases by up to 100-fold with distance from the fovea.

Cone cells in the fovea are the primary targets of gene therapies aiming to treat inherited retinal diseases like retinitis pigmentosa. Usually, viral vectors encoding therapeutic proteins are injected "subretinally", i.e. into the subretinal space between the photoreceptors and the retinal pigment epithelium (RPE) cells in order to provide gene delivery to cones.

The subretinal delivery leads to the formation of a "bleb", which refers to a fluid-filled pocket within the subretinal space of the injected eye. In this approach, gene delivery is limited to cells that contact the local bleb of injected fluid. Retinal detachment, and in particular foveal detachment, that occurs during subretinal injections is a concern in eyes with retinal degeneration.

Advantageously, when the vector is an AAV9-7m8 vector (in particular AAV9-7m8-pR1.7 vector), the vector (or carrier of pharmaceutical composition comprising said vector) can be administered by a distal subretinal injection, or in the periphery of the fovea, and then spread laterally to reach the foveal region. According to an embodiment the bleb is formed greater than or equal to 0.5 millimeters away from the center of the fovea, without detaching the foveal region. In an embodiment, said AAV9-7m8 viral vector is formulated in a solution and 50 to 100 µL of solution are injected continuously in 20 to 30 seconds. In an embodiment, said AAV9-7m8 viral vector is formulated in a solution at a concentration of 1×10¹⁰ to 1×10¹² vg/mL (viral genome/mL), preferably of 0.5×10¹¹ to 5×10¹¹ vg/mL, still preferably of 1×10¹¹ vg/mL. Preferably, the cone precursor cells are administered by intraocular injection, preferably by subretinal space injection, more preferably by injection between the neural retina and the overlying PE. The amount of cone precursor cells to be administered may be determined by standard procedure well known by those of ordinary skill in the art. Physiological data of the patient (e.g. age, size, and weight) and type and severity of the disease being treated have to be taken into account to determine the appropriate dosage. The cone precursor cells may be administered as a single dose or in multiple doses. In particular, each unit dosage may contain, from 100,000 to 300,000 cone precursor cells per µl, preferably from 200,000 to 300,000 cone precursor cells per µl.

### Brief description of the figures

**Figure 1** represents phototransduction cascade (A) normal phototransduction cascade (B) short phototransduction cascade with an animal opsin and GIRK2 channel. PDE: phosphodiesterase. CNG: cyclic-nucleotic gated channels. cGMP: cyclic guanosine monophosphate.

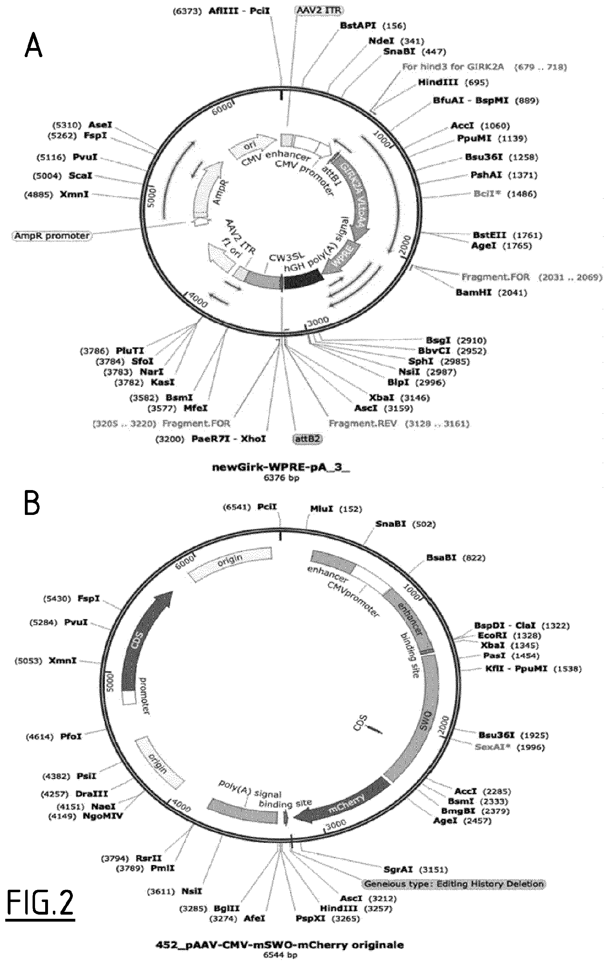

**Figure 2** represents plasmids (A) CMV-GIRK2-GFP and (B) CMV-SWO-mCherry.
**Figure 3** represents what remained in the phototransduction cascade in rd10 mice using immunohistochemistry (A-D) retinal cross-section of a control WT mouse stained with (A) opsin, (B) transducin, (C) PDE and (D) cone arrestin. (E-H) retinal cross-section of a rd10 mouse at P14 stained with (E) opsin, (F) transducin, (G) PDE and (H) cone arrestin. (I-L) Retinal cross-section of a rd10 mouse at P150 stained with (I) opsin, (J) transducin, (K) PDE and (L) cone arrestin. ONL: outer nuclear layer. INL: inner nuclear layer. GC: ganglion cells. Scale bar is 50µm. Inset scale bar is 25µm.

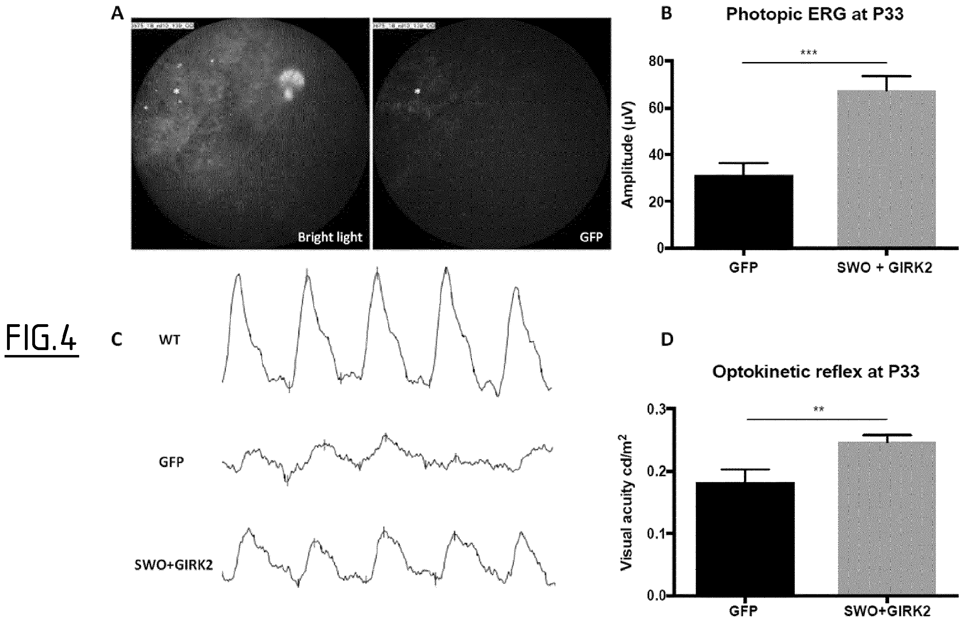

**Figure 4** represents preliminary data. (A) Eye fundus of GIRK2-GFP expression in rd10 mouse one week post-injection *(*site of injection)* (B) Photopic ERG amplitude in rd10 mice at P33, injected with AAV-SWO-tdTomato and AAV-GIRK2-GFP. Control mice are injected with AAV-GFP (n=12). *P=0,0002.* (C) Representative flickers ERG at P33. (D) Measure of the visual acuity by optokinetic test in rd10 mice, injected with AAV-SWO-tdTomato and AAV-GIRK2-GFP. Control mice are injected with AAV-GFP. Control mice were injected with AAV-GFP (n=8).

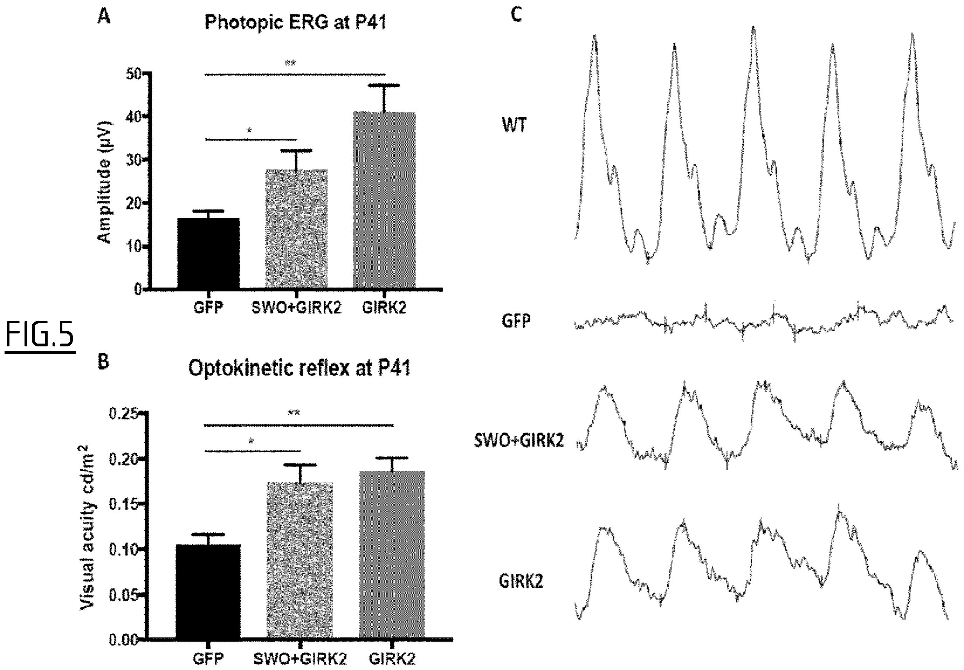

**Figure 5** represents GIRK2-mediated vision. (A) Photopic ERG amplitude in rd10 mice at P41, injected with AAV-SWO-tdTomato and/or AAV-GIRK2-GFP. Control mice are injected with AAV-GFP (n=12). *P_{SWO+GIRK2}= 0,0381 and P_{GIRK2}=0,0021.* (B) Measure of the visual acuity by optokinetic test in rd10 mice, injected with AAV-SWO-tdTomato and/or AAV-GIRK2-GFP. Control mice are injected with AAV-GFP. Control mice were injected with AAV-GFP (n=7). (C) Representative flickers ERG at P41.

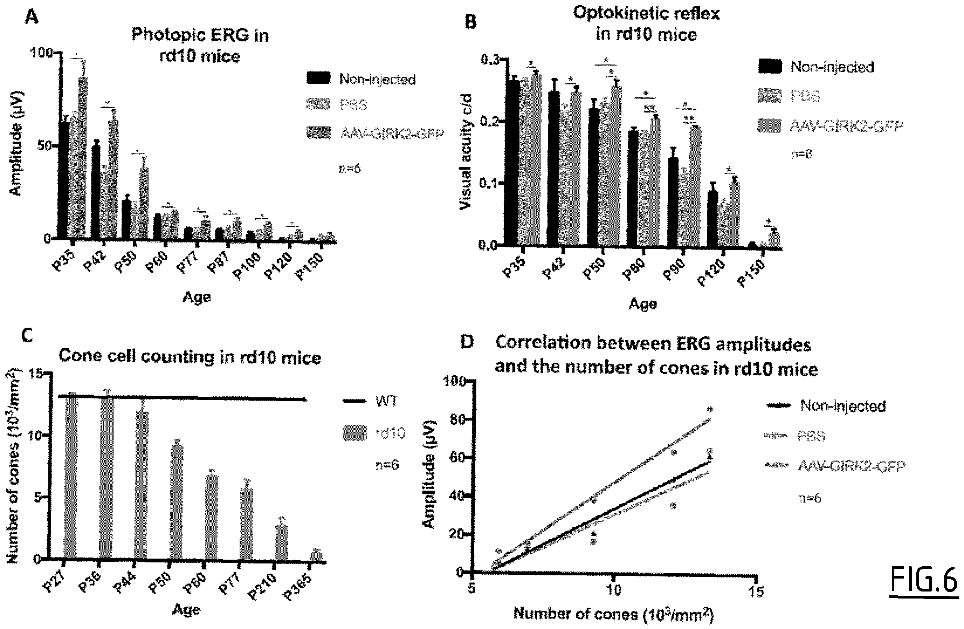

**Figure 6** represents long term efficiency. (A) Photopic ERG amplitude in rd10 mice, injected with AAV-GIRK2-GFP. Control mice are injected with PBS (n=6). (B) Measure of the visual acuity by optokinetic test in rd10 mice, injected with AAV-GIRK2-GFP. Control mice are injected with PBS (n=6). (C) Number of cones of wild-type mice and non-injected rd10 mice over time (n=6). *Pvalue _{(P50-P365)} = 0.0022.* (D) Linear regression correlation between the ERG amplitudes and the number of cones in rd10 mice (n=6). *Pvalue _{non-injected} = 0,0482. Pvalue _{AAV-GIRK2-GFP} = 0,0007. Pvalue _{PBS} = 0,0104.*

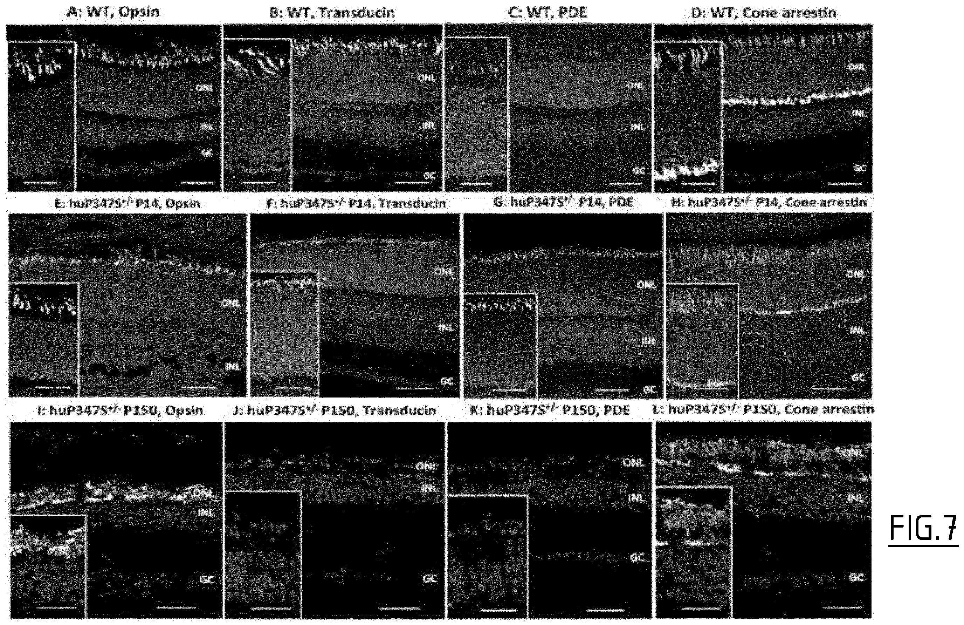

**Figure 7** represents what remained in the phototransduction cascade in huP347S^{+/-}mice using immunohistochemistry. (A-D) Retinal cross-section of a control WT mouse stained with (A) opsin, (B) transducin, (C) PDE and (D) cone arrestin. (E-H) retinal cross-section of a huP347S^{+/-} mouse at P14 stained with (E) opsin, (F) transducin, (G) PDE and (H) cone arrestin. (I-L) retinal cross-section of a huP347S^{+/-} mouse at P150 stained with (I) opsin, (J) transducin, (K) PDE and (L) cone arrestin. ONL: outer nuclear layer. INL: inner nuclear layer. GC: ganglion cells. Scale bar is 50µm. Inset scale bar is 25µm.

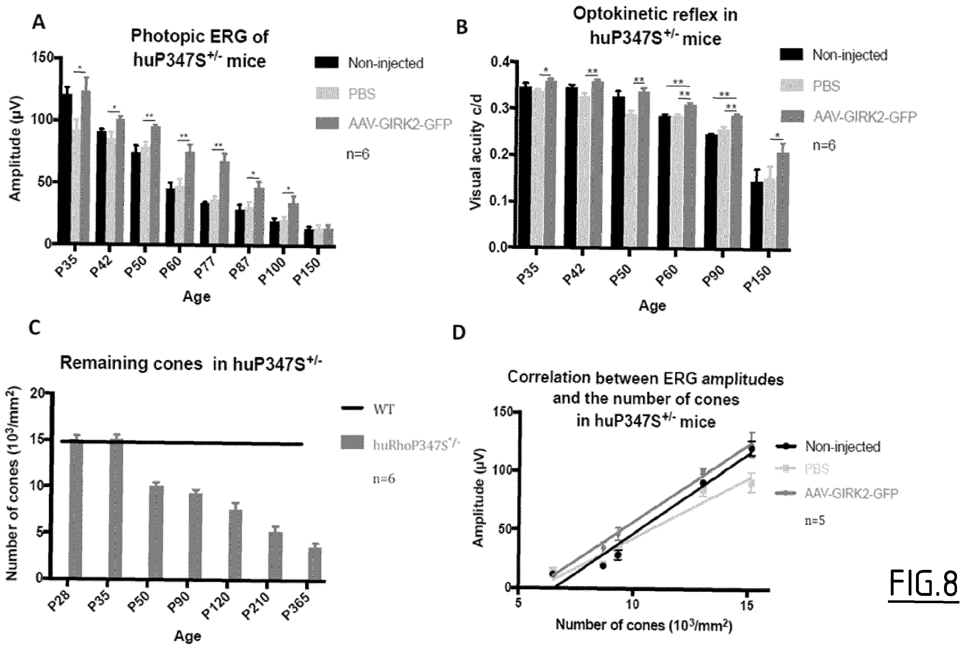

**Figure 8** represents universality of the approach. (A) Photopic ERG amplitude in huP347S^{+/-} mice, injected with AAV-GIRK2-GFP. Control mice are injected with PBS (n=6). (B) Measure of the visual acuity by optokinetic test in huP347S^{+/-} mice, injected with AAV-GIRK2-GFP. Control mice are injected with PBS (n=6). (C) Number of cones of wild-type mice and non-injected huP347S^{+/-} mice over time (n=6). *Pvalue _{(P50-P365)} = 0,0022.* (D) Linear regression correlation between the ERG amplitudes and the number of cones in huP347S^{+/-} mice (n=5). *Pvalue _{non-injected} = 0, 0313. Pvalue _{AAV-GIRK2-GFP} = 0,0146. Pvalue _{PBS} = 0,0497.*

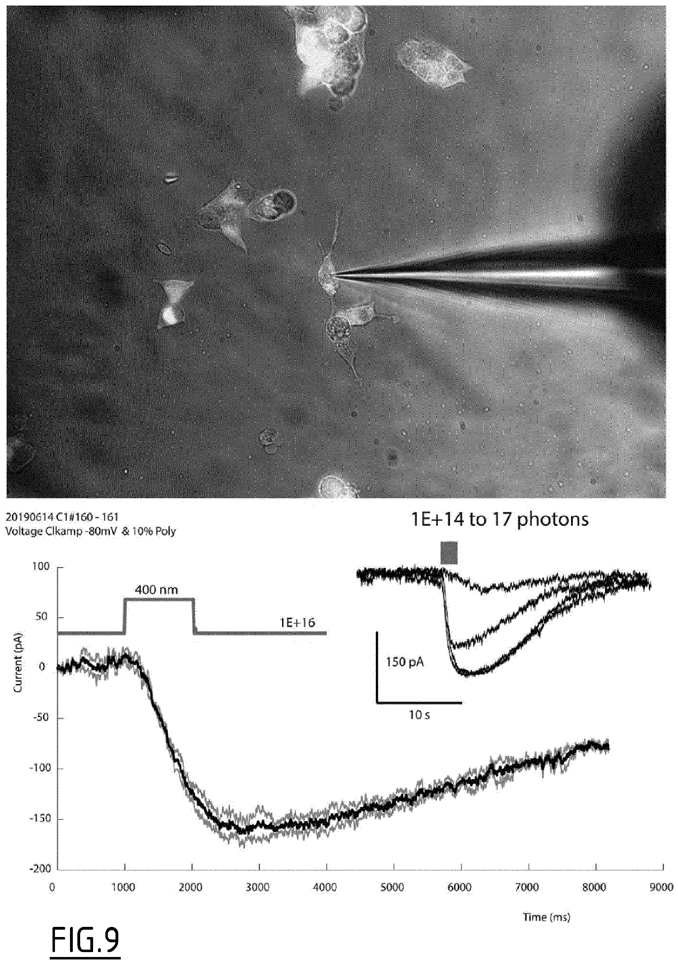

**Figure 9** represents the efficiency of the mouse GIRK2 in HEK cells transfected with two plasmids: CMV-SWO-mCherry and CMV-GIRK2-GFP.
**Figure 10** represents phenotyping of a normal volunteer and retinitis pigmentosa patients for eligible patient population. Upper panel (A) shows the fundus and OCT images of the back of the eye in a normal individual along with adaptive optics images of cone dominated regions of the retina. Middle panel (B) shows a pie-chart distribution of advanced RCD patients. Lower panels represent OCT and AOSLO images of different patients. AOSLO confocal (up) and AOSLO split detection (low) in vivo retinal images of a patient with retinitis pigmentosa (age 77, male). Acquired fields are located at the transition between regions of presumed dormant cones (i.e. morphologically intact - as indicated by the IS/OS line visible in OCT, clear inner segment mosaic visible in AOSLO split detection, and clear cone mosaic indicating intact inner and outer segments in AOSLO confocal - though with reduced function according to the patient's visual acuity; yellow bars) and damaged or absent cones (indicated by the absent IS/OS line in OCT, and the blurred inner segment and cone mosaics in AOSLO split detection and confocal respectively; red bars). Yellow arrows indicate cones that appear to be degenerating, with absent OS in confocal but present IS in split detection. Scale bars, 200µm.
**Figure 11** represents cone opsin and arrestin expression in normal and RCD human retinal tissue. (A) retinal cross-section of a 91-year-old individual with no visual impairment (40x). 5B-E) Retinal cross-sections of human RCD maculae from 4 different donors (40x).

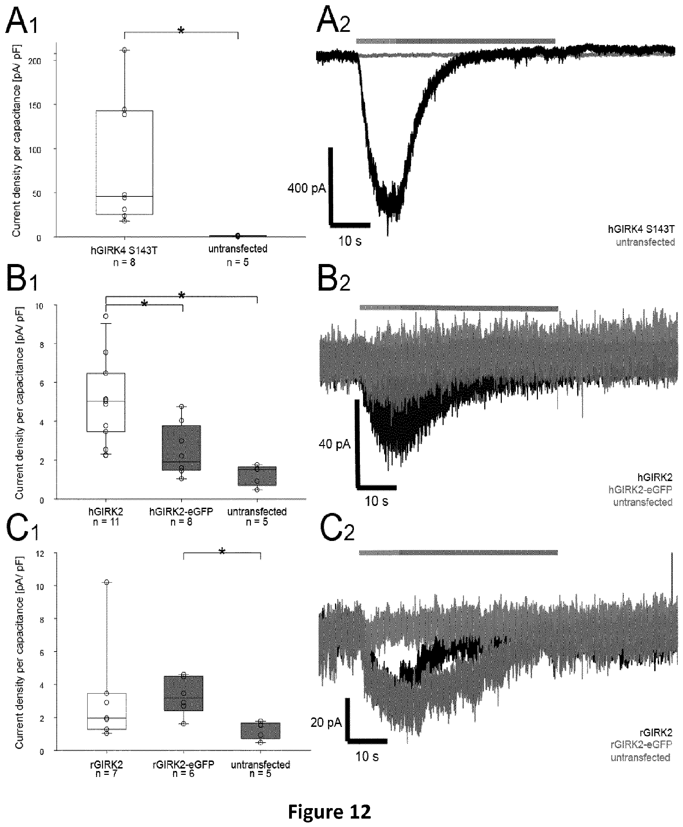

**Figure 12****:** shows the current density per capacitance of HEK293 cells expressing **A1)** hGIRK4 S143T, **B1)** hGIRK2 or **C1)** truncated rGIRK2. The current response, elicited by the activation of mOpn4L, is shown in comparison to constructs with c-terminal eGFP fusion and untransfected cells. The current density of individual cells is illustrated by individual data points (circles). Significant differences between conditions are marked with * (Kruskal-Wallis One Way Analysis of Variance on Ranks with all pairwise multiple comparison procedures (Dunn's Method) p<0.05) When activated with blue light mOpn4L induces a GIRK-mediated current that concludes upon stimulation with lime light as shown in exemplary traces of **A2)** hGIRK4 S143T, **B2)** hGIRK2 and **C2)** truncated rGIRK2 constructs.

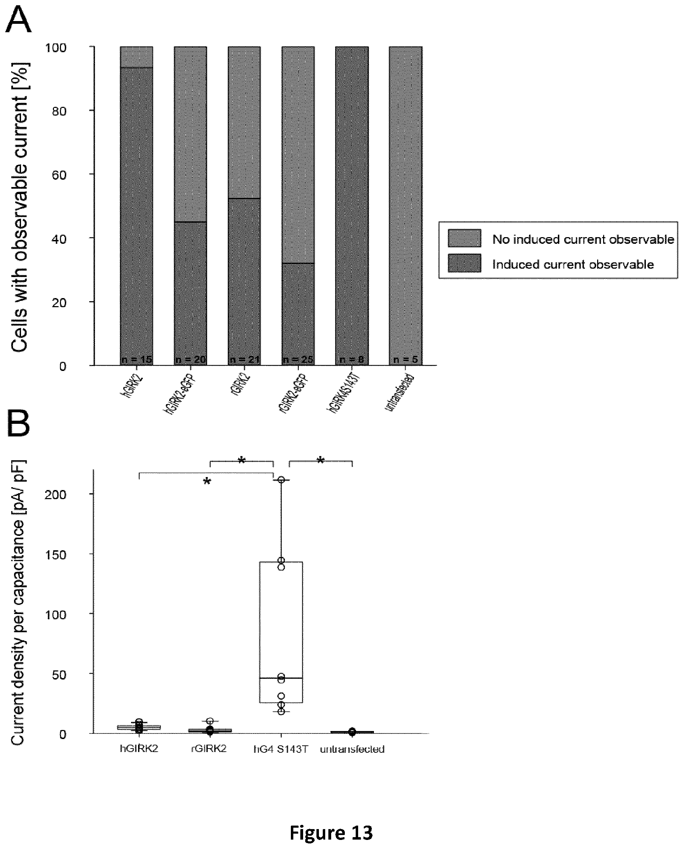

**Figure 13 A)** Stimulation of HEK293 cells expressing mOpn4L and either hGIRK4 S143T or hGIRK2 produced a reliably observable current in whole-cell patch clamp recordings, while currents of hGIRK2-eGFP, rGIRK2 or rGIRK2-eGFP after mOpn4L activation were less likely to be observed. Results of "no induced current observable" are provided in the upper part of each graph bar. Results of "Induced current observable" are provided in the lower part of each graph bar. **B)** Current density per capacitance of HEK293 cells expressing the different GIRK constructs (top) or their eGFP tagged versions (bottom). The current density of individual cells is illustrated by individual data points (circles).

### EXAMPLES

### EXAMPLE 1 : MATERIAL AND METHODS

### 1. Animals

C57BL/6j^{rd10/rd10} (rd10) mice were used in these experiments. They have a mutation on the rod PDE gene leading to a dysfunctional phototransduction cascade and a rod-cone dystrophy. The second model used is the huRhoP347S^{+/-} mouse. The homozygous strand of this mouse present a KO of mouse rhodopsin (mRho) gene and a KI of human rhodopsin (huRho) with a mutation (P347S) (Millington-Ward et al., 2011) [30]. The homozygous males were crossed C57BU6j (wild-type) females to obtain heterozygous mice. These mice have a similar phenotype as the rd10 mice but the degeneration rate is lower.

### 2. AAV injections

Mice were first anesthetised with intraperitoneal injections of 0.2 ml/20g ketamine (Ketamine 500, Virbac France) and xylazine (Xylazine 2%, Rompun) diluted in 0.9% NaCl. Eyes were dilated with 8% Neosynephrine (Neosynephrine Faure 10%, Europhta) and 42% Mydriaticum (Mydriaticum 0.5%, Thea) diluted in 0.9% NaCl.

A total volume of 1 µl of vector solution was injected subretinally. Fradexam, an ophthalmic ointment, was applied after injection. The list of injected viral vectors is presented below:

| Injection Table | | | | |
|---|---|---|---|---|
| Mice | Eyes | viral vector injected | viral vector titration | volume injected |
| rd10 | both | AAV8-mCAR-GFP | 10¹¹ rAAV particles | 1 µl for all conditions |
| rd10 | both | AAV8-mCAR-GIRK2-GFP+ AAV8-mCAR-SWO-tdTomato | | |
| rd10 | both | AAV8-mCAR-GIRK2-GFP | | |
| rd10 | right | PBS | | |
| rd10 | left | AAV8-PR1.7-GIRK2-GFP | 5.10⁸ rAAV particles | |
| huRhoP347S^{+/-} | right | PBS | | |
| huRhoP347S^{+/-} | left | AAV8-PR1.7-GIRK2-GFP | 5.10⁸ rAAV particles | |

### 3. Eye fundus examination

One week after subretinal injection, mice were anesthetised by isofluorane inhalation. Eyes were dilated and then protected with Lubrithal eye gel (VetXX). Fundus imaging was performed with a fundus camera (Micron III; Phoenix research Lab) equipped with specific filters to monitor GFP or tdTomato expression in live anesthetised mice.

### 4. Electroretinography (ERG) recordings

To evaluate retinal function, electroretinography recordings (ERG) were recorded (espion E2 ERG system; Diagnosys). Several tests were performed at different time points after injections of the viral vectors. Mice were anesthetised with intraperitoneal injections of 0.2 ml/20g ketamine (Ketamine 500, Vibrac France) and xylazine (Xylasine 2%, Rompun) diluted in 0.9% NaCl. Mice were then placed on a heated pad at 37°C. Eyes were dilated with Neosyhephrine (Neosynephrine Faure 10%, Europhta) and Mydriaticum (Mydriaticum 0.5%, Thea) diluted in 0.9% NaCl. Eyes were protected with Lubrithal eye gel before putting electrodes on the corneal surface of each eye. The reference electrode was inserted under the skin into the forehead and a ground electrode under the skin in the back.

ERG recordings were done under two conditions: (i) photopic condition, which reflects con-driven light responses - 6ms light flashes were applied every second during 60 seconds at increasing light intensities (0.1/1/10/50cd s/m) after an adaptation of 5 minutes at 20cd s/m - and (ii) flicker condition, which are rapid frequency light stimuli that reflect cone function (70 flashes at 10Hz et 1cd s/m).

Graph and statistical analysis were performed using GraphPad.

### 5. Optokinetic test

Visual acuity was measured using an optokinetic test scoring the head turning movement of a mouse placed in front of moving bars. Testing was performed using a computer-based machine consisting of four computer monitors arranged in a square to form an optokinetic chamber. A computer program was designated to generate the optokinetic stimuli, consisting of moving alternate black and white stripes. The spatial frequency is ranging from 0.03 to 0.6 cyc/deg. The program enabled modulation of stripe width and direction of bar movement.

### 6. Immunohistochemistry and confocal imaging

Animals were sacrificed by CO₂ inhalation, and the eyes were enucleated and fixed in 4% paraformaldehyde-PBS for 1h at room temperature. The eyes were dissected either as eyecups for immunohistochemistry or prepared as flat mounts for cell counting. Human retinal samples were obtained from the Foundation Fighting Blindness (USA) and were fixed with a mix of paraformaldehyde and glutaraldehyde. Mouse eyecups and human retinas were cryoprotected with a gradient of PBS-Sucrose 10% for 1h and then in PBS-Sucrose 30% overnight, then embedded in Optimal Cutting Temperature (OCT) compound. The 12µm thick cryostat sections were cut and mounted on glass slides. Sections were stained with primary and secondary antibodies (Supplementary Table 2) and DAPI (1:2000). The sections were finally washed in PBS, mounted in Fluoromount Vectashield (Vector Laboratories) and cover-slipped for imaging using a laser-confocal microscopy (Olympus IX81). For flat-mount retina stainings, the protocol is the same except that the tissue was not cryoprotected. Images were analysed using FIJI software.

| Antibody table | | |
|---|---|---|
| Target | Host | Clonality/Conjugated |

| PRIMARY ANTIBODIES | | |
|---|---|---|
| Red/Green opsin (M/L opsin) | Rabbit | Polyclonal |
| Mouse Cone arrestin | Rabbit | Polyclonal |
| PDE6C | Rabbit | Polyclonal |
| GNAT2 | Rabbit | Polyclonal |
| PNA-Lectin | Conjugated with FITC | |

| SECONDARY ANTIBODIES | | |
|---|---|---|
| Anti-rabbit | Donkey | AF 546 |

### 7. Cell counts

Flat mount retinas of rd10 and huRhoP347S^{+/-} mice were stained using antibodies against mouse cone arrestin - mCAR (1:10000) and DAPI (1:2000). The double stained cells counted at different ages. Retinas from 5 animals (n=10) were used for each age and were oriented dorso-ventrally and naso-temporally. Serial optical sections were obtained to cover the thickness of the entire outer nuclear layer (ONL). Two scanning areas of 211.97 × 211.97 µm were made in each of the four regions in all retinas. Counts of cone cells were performed manually using the FIJI software by the reconstruction of the images (z stack) covering the entire thickness of the ONL. Average density values of each retina were calculated to obtain the number of cone cells per mm² at different ages.

### 8. In vitro test of the efficiency of mouse GIRK2

HEK cells were transfected with two plasmids: CMV-SWO-mCherry and CMV-GIRK2-GFP (Figure 2) according to a well-known procedure in the art. HEK293 cells were cultured and recorded in dark room conditions after transfection. Cells were placed in the recording chamber of a microscope equipped with a 25x water immersion objective (XLPlanN-25 _{×} -W-MP/NA1.05, Olympus) at 36 °C in oxygenated (95% O2/5% CO2) Ames medium (Sigma-Aldrich) enriched with an addition of 1 mM9-cis-retinal. KGluconate was added to the external solution in order to get a high extracellular potassium concentration leading to a cell potassium reversal potential of -40mV.

For Whole-cell recordings, the Axon Multiclamp 700B amplifier (Molecular Device Cellular Neurosciences) was used, GIRK-mediated K+-currents were recorded in voltage-clamp configuration at -80 mV, using borosilicate glass pipettes (BF100-50-10, Sutter Instrument) pulled to 5MΩ and filled with 115 mMK Gluconate, 10 mM KCI, 1 mM MgCl2, 0.5 mM CaCl2, 1.5 mM EGTA, 10 mM HEPES, and 4 mM ATP-Na2 (pH 7.2).

During experiments, a CCD camera (Hamamatsu Corp.) was used to visualize cells using a trans-illuminated infrared-light. A monochromatic light source (Polychrome V, TILL photonics) was used to stimulate cells during electrophysiological experiments with light flashes at 400 nm.

### 9. Patient eye fundus imaging

Adaptive optics scanning laser ophthalmoscopy (AOSLO) (Roorda et al Opt Exp 2002) was used to image cone photoreceptor mosaic at cell resolution. The AOSLO device used (MAORI, PSI, Andover, MA, USA) allows simultaneous imaging over a 2-degree field of view of intact cones with both inner and outer segments (IS, OS) from light scattered along the optical axis (confocal mode) and inner segments (IS) from multiply scattered light scattered off axis (split detection mode). This allows us to evaluate cone presence and health, with differential imaging of IS versus IS+OS for each cone.

### EXAMPLE 2: RESULTS

### 1. The changes in the phototransduction cascade in degenerating cones

The phototransduction cascade was first analysed in the rd10 mouse model by studying its components using immunohistochemistry, at different time points during retinal degeneration. Immunofluorescence staining was performed against cone opsin, transducing, phosphodiesterase and cone arrestin proteins of the phototransduction cascade that interact directly with cone opsin.

Figure 3 shows that only the cone opsin and arrestin were still expressed and localized around the cone cell body at late stage of the disease.

### 2. Cone opsin and GIRK2-mediated vision restoration

Based on immunohistochemistry and previous findings with cone opsins expressed in neurons, it was first studied why delivering a mouse short wavelength cone opsin (SWO) fused with tdTomato and rat truncated GIRK2 fused with GFP using two AAV vectors mixed in equimolar ratios would enhance cone cell's response to light. Thus two AAVs were injected subretinally to degenerating rd10 mouse retinas at p15 (Figure 4A). This led to a significant increase in phototpic ERG amplitudes in treated eyes compared to controls (Figure 4B). Flicker ERGs confirmed that the recovery mechanism was still active in these cone cells expressing GIRK2 allowing them to follow a fast stimulus (Figure 4C). The rd10 animals treated with GIRK2 showed also an improved optokinetic reflex compared to controls (Figure 4D).

Next it was studied if the endogenous cone opsin, still present in degenerating cones, was functional and sufficient to activate GIRK2 channel in this mouse model. For this, a single AAV8 vector encoding GIRK2 in fusion with GFP was delivered. This led to similar increases in photopic ERG amplitudes and optokinetic reflex in treated eyes compared to controls confirming that GIRK2 alone was sufficient to increase light sensitivity via G protein coupled signalling involving cone opsin (Figure 5A-B). Flicker ERGs were also robustly amplified with this approach (Figure 5C).

### 3. GIRK2-mediated vision restoration: long-term efficacy

Photopic ERG recordings were performed to monitor the cone response to light stimuli at different time points after treatment with GIRK2 and in absence of treatment. These ERGs were done under two conditions: (i) photopic with light flashes applied every second during 60 seconds at increasing light intensities and (ii) flicker stimulation with repetitive flashes during 60 seconds. Data were collected on a weekly basis until p50 and then every 10 to 13 days until 11 weeks of age and showed a gradual decline in ERG amplitudes for both controls and treated eyes (Figure 6A). Moreover, these results are consistent with the optokinetic test, both controls and treated eyes with GIRK2 show a decreased optokinetic reflex over time (Figure 6B). This decline was to be expected as cone numbers also decreased over time in the rd10 mice (Figure 6C). The number of cone photoreceptors remaining in rd10 retinas were counted to correlate decreases in cone numbers with decrease in ERG amplitudes. Indeed, the decrease in light responses was proportional to number of remaining photoreceptors (Figure 6D). It was thus concluded that GIRK2 increased light responses in remaining cones so long as cones remained alive but, as expected, it did not slow down the loss of cone cells.

### 4. GIRK2-mediated vision restoration in an RCD model caused by mutant rhodopsin

Having in mind the goal of creating a mutation-independent therapy, the approach was tested in another mouse model - with a different causal mutation. To this aim experiments were done in a heterozygous mouse model called mRho^{-/-}huRhoP347S^{+/-} carrying a knock in for P347S mutant human rhodopsin. Mutant human rhodopsin and absence of mouse rhodopsin led to a rod-cone dystrophy in this complementary model. Here, the same set of experiments was repeated as that was done in the rd10 mouse model. First, the phototransduction cascade proteins interacting with cone opsin at different time points was analysed (Figure 7). It was noticed that: (i) the degeneration rate was slower compared to rd10 and (ii) similar to the rd10 model only the opsin and the arrestin persisted in cone cell bodies at P150.

Next, mice were injected at P15 with the same AAV vectors encoding for GIRK2 fused with GFP and recorded ERGs to monitor cone response to light stimuli at various time points (Figure 8A). The response amplitudes of treated eyes were significantly higher than that of control eyes until P100. Moreover, flicker ERG responses were also similarly improved in this mouse model. Similarly to rd10 mice, this mouse model also shows an improved optokinetic reflex that decreases over time in both control and treated conditions (Figure 8B). This decline is to be expected as cone numbers also decreases over time in this RCD mouse model (Figure 8C). The decrease in time in ERG amplitudes also correlated with a decrease in cone numbers in this model (Figure 8D). This was again consistent with the fact that the approach did not stop the degeneration but allowed for enhanced light sensitivity via GIRK2.

### 5. Efficiency of the mouse GIRK2 in an in vitro test

Light stimulations (400nm, 5 seconds, fullfield) activated GIRK currents in HEK cells expressing both GIRK and SWO (Short Wavelength Opsin) (Figure 9). GIRK channels are modulated in a membrane-delimited, fast manner via the Gi/o pathway and the expression of the mouse GIRK channel was membrane bound. The amplitudes and kinetics of light-induced activation and deactivation of GIRK channels with SWO, induce large GIRK current amplitudes during a 5 s light pulse.

### EXAMPLE 3: GIRK4 S143T induces significant ion efflux, as compared to either human or rat GIRK2

### 1. Creating a stable cell line for the characterization of the GIRK constructs

A cell line stably expressing mOpn4L.mCherry was ordered from and created by VectorBuilder. This cell line expresses mouse melanopsin (mOpn4L) tagged with mCherry and allows for the activation of the G_{i/o} pathway in response to blue light. The cell line will be used to analyze the GIRK constructs to be examined.

### 2. In-vitro assessment of GIRK-currents

For the assessment of G-protein-gated inwardly rectifying potassium channel (GIRK) currents plasmids coding for human GIRK2 or GIRK4 as well as truncated rat GIRK2, proteins above have been expressed in human embryonic kidney cells (HEK293) stably expressing mouse melanopsin (mOpn4L) tagged with mCherry. Under the control of a CMV promotor expression was either visualized by eGFP fusion or by co-expression of a separate construct coding solely for eGFP.
HEK293 cells stably expressing mOpn4L-mCherry were maintained in Dulbecco's modified Eagle's medium (4.5 g/l D-glucose, Sigma-Aldrich) supplemented with 10% fetal bovine serum (Gibco), 1% penicillin-streptomycin (Gibco) and 1.5 pg/ml puromycin (Gibco) at 37 °C and 5% CO₂ in a humidified incubator. The same protocol was used for the treatment of tsA201 but without the use of puromycin. Cells were transfected with FuGENE^{®} HD (Promega) according to the manufacturer's protocol 12 - 24 h before recordings and then shielded from light until recorded. After exchanging the growth medium for an external solution (20 mM NaCl, 120 mM KCI, 2 mM CaCl₂, 1 mM MgCl₂, 10 mM HEPES, pH 7.3 (KOH)) and application of 1 µM retinal whole-cell patch clamp recordings were carried out with patch pipettes (2 - 4 MΩ, MPC325, Sutter Instrument) filled with an internal patch solution (100 mM L-Aspartic acid potassium, 40 mM KCI, 40 mM MgATP, 10 mM HEPES, 5 mM NaCl, 2 mM EGTA, 2 mM MgCl₂, 0.01 mM GTP, pH 7.3 (KOH)) and an USB-amplifier (EPC10, Heka). Currents were digitized and filtered with an internal 10-kHz three-pole Bessel filter (filter 1) in series with a 2.9-kHz 4-pole Bessel filter (filter 2) and monitored by PatchMaster (v2X52, HEKA), which also served to control voltage and a monochromator for the stimulation of mOpn4L or OPN1LW (Polychrome V, Till Photonics). Cells expressing mOpn4L and individual GIRK constructs were voltage clamped at -60 mV and their baseline current recorded for 10 s. Activation of mOpn4L with a 10 s light pulse (471 nm) allowed for the recording of GIRK mediated current, which concluded within a deactivating stimulation period with lime light (560 nm) for 40 s. The same voltage clamp protocol and a 10 s light pulse (560 nm) were applied to verify GIRK currents elicited by OPN1LW. The peak electrical current density per capacitance at HEK membranes during activation of mOpn4L or OPN1LW was then assessed and compared with Igor Pro 6.11 (WaveMetrics) and SigmaPlot 12.5 (Systat) and visually processed with the help of CorelDRAW 2018 (Corel).

### List of references

1. Sinha R, Hoon M, Baudin J, Okawa H, Wong ROL, Rieke F. 2017. Cellular and Circuit Mechanisms Shaping the Perceptual Properties of the Primate Fovea. Cell. 168(3):413-426.e12
2. Yau KW, Hardie RC. 2009. Phototransduction motifs and variations. Cell. 139(2):246-64
3. Ebrey T, Koutalos Y. 2001. Vertebrate photoreceptors. Prog Refin Eye Res. 20(1):49-94
4. Larhammar D, Nordström K, Larsson T a. 2009. Evolution of vertebrate rod and cone phototransduction genes. Philosophical transactions of the Royal Society of London. Series B, Biological sciences. 364(1531):2867-80
5. Maeda T, Imanishi Y, Palczewski K. 2003. Rhodopsin phosphorylation: 30 Years later. Prog. Retin Eye Res. 22(4): 417-434
6. Buch H, Vinding T, La Cour M, Appleyard M, Jensen GB, Nielsen NV. 2004. Prevalence and Causes of Visual Impairment and Blindness among 9980 Scandinavian Adults: The Copenhagen City Eye Study. Ophthalmology. 111(1):53-61
7. Wright AF, Chakarova CF, Abd El-Aziz MM, Bhattacharya SS. 2010. Photoreceptor degeneration: genetic and mechanistic dissection of a complex trait. Nature reviews. Genetics. 11(4):273-84
8. Ferrari S, Di Iorio E, Barbaro V, Ponzin D, Sorrentino FS, Parmeggiani F. 2011. Retinitis pigmentosa: genes and disease mechanisms. Current genomics. 12(4):238-49
9. Li ZY, Kljavin IJ, Milam a H. 1995. Rod photoreceptor neurite sprouting in retinitis pigmentosa. The Journal of neuroscience: the official journal of the Society for Neuroscience. 15(8):5429-38
10. Bennett J. 2017. Taking Stock of Retinal Gene Therapy: Looking Back and Moving Forward. Molecular Therapy. 25(5):1076-94
11. Dalkara D, Duebel J, Sahel J-A. 2015. Gene therapy for the eye focus on mutation-independent approaches. Current Opinion in Neurology. 28(1):51-60
12. Busskamp V, Picaud S, Sahel JA, Roska B. 2012. Optogenetic therapy for retinitis pigmentosa. Gene Therapy. 19(2):169-75
13. Dalkara D, Sahel J-A. 2014. Gene therapy for inherited retinal degenerations. C. R. Biol. 337(3): 185-192
14. Scholl HPN, Strauss RW, Singh MS, Dalkara D, Roska B, et al. 2016. Emerging therapies for inherited retinal degeneration. Science Translational Medicine. 8(368):368rv6-368rv6
15. Baker CK, Flannery JG. 2018. Innovative Optogenetic Strategies for Vision Restoration. Frontiers in Cellular Neuroscience. 12(September):1-8
16. Cehajic-Kapetanovic J, Eleftheriou C, Allen AE, Milosavljevic N, Pienaar A, et al. 2015. Restoration of Vision with Ectopic Expression of Human Rod Opsin. Current Biology. 25(16):2111-22
17. Gaub BM, Berry MH, Holt AE, Isacoff EY, Flannery JG. 2015. Optogenetic Vision Restoration Using Rhodopsin for Enhanced Sensitivity. Molecular therapy: the journal of the American Society of Gene Therapy. 23(10):1562-71
18. Van Gelder RN, Kaur K. 2015. Vision Science: Can Rhodopsin Cure Blindness. Current Biology. 25(16):R713-15
19. van Wyk M, Pielecka-Fortuna J, Löwel S, Kleinlogel S. 2015. Restoring the ON Switch in Blind Retinas: Opto-mGluR6, a Next-Generation, Cell-Tailored Optogenetic Tool. PLOS Biology. 13(5):e1002143
20. Berry MH, Holt A, Levitz J, Visel M, Aghi K, et al. Restoration of high-sensitivity , adapting , patterned vision with a cone opsin. Nature Communications. (2019):1-12
21. De Silva SR, Barnard AR, Hughes S, Tam SKE, Martin C, et al. 2017. Long-term restoration of visual function in end-stage retinal degeneration using subretinal human melanopsin gene therapy. Proceedings of the National Academy of Sciences. 114(42):11211-16
22. Lin B, Koizumi A, Tanaka N, Panda S, Masland RH. 2008. Restoration of visual function in retinal degeneration mice by ectopic expression of melanopsin. Proceedings of the National Academy of Sciences of the United States of America. 105(41):16009-14
23. Masseck OA, Spoida K, Dalkara D, Maejima T, Rubelowski JM, et al. 2014. Vertebrate Cone Opsins Enable Sustained and Highly Sensitive Rapid Control of G i/o Signaling in Anxiety Circuitry. Neuron. 81(6):1263-73
24. Mark MD, Herlitze S. 2000. G-protein mediated gating of inward-rectifier K+ channels. Eur. J. Biochem. 267(19): 5830-5836
25. Busskamp V, Duebel J, Balya D, Fradot M, Viney TJ, et al. 2010. Genetic Reactivation of Cone Photoreceptors Restores Visual Responses in Retinitis Pigmentosa. Science. 329(5990):413-17
26. Packer AM, Roska B, Häusser M. 2013. Targeting neurons and photons for optogenetics. Nature neuroscience. 16(7):805-15
27. Khabou H, Garita-Hernandez M, Chaffiol A, Reichman S, Jaillard C, et al. 2018. Noninvasive gene delivery to foveal cones for vision restoration. JCI Insight. 3(2):1-18
28. Demande internationale WO 2018134168
29. Byrne LC, Dalkara D, Luna G, Fisher SK, Clérin E, et al. 2015. Viral-mediated RdCVF and RdCVFL expression protects cone and rod photoreceptors in retinal degeneration. Journal of Clinical Investigation. 125(1):105-16
30. Millington-Ward S, Chadderton N, O'Reilly M, Palfi A, Goldmann T, et al. 2011. Suppression and Replacement Gene Therapy for Autosomal Dominant Disease in a Murine Model of Dominant Retinitis Pigmentosa. Molecular Therapy. 19(4):642-49
31. Kim W. Chan, Jin-Liang Sui, Michel Vivaudou, Diomedes E. Logothetis, 1996, Control of channel activity through a unique amino acid residue of a G protein-gated inwardly rectifying K+ channel subunit, Proc Natl Acad Sci USA. ; 93(24): 14193-14198
32. Vivaudou M, Chan KW, Sui JL, Jan LY, Reuveny E and Logothetis DE, 1997, Probing the G-protein regulation of GIRK1 and GIRK4, the two subunits of the KACh channel, using functional homomeric mutants. Journal of biological chemistry. Vol. 272, No 50, pp.31553-31560
33. Ye et al., 2016, Cone-Specific Promoters for Gene Therapy of Achromatopsia and Other Retinal Diseases. Hum Gene Ther. Jan;27(1):72-82.
34. Dalkara et al., 2013. In Vivo-Directed Evolution of a New Adeno-Associated Virus for Therapeutic Outer Retinal Gene Delivery from the Vitreous. SCIENCE TRANSLATIONAL MEDICINE, Vol 5, Issue 189, p. 189ra76
35. Byrne et al. 2020, In vivo-directed evolution of adeno-associated virus in the primate retina. JCI Insight. 5(10)
36. Kotterman et al. 2021, Directed Evolution of AAV Targeting Primate Retina by Intravitreal Injection Identifies R100, a Variant Demonstrating Robust Gene Delivery and Therapeutic Efficacy in Non-Human Primates. BioRxiv.

## Claims

1. A vector comprising a nucleotide sequence encoding a S143T mutated form of the subunit 4 of G-protein-gated inwardly rectifying potassium channel (GIRK4) (GIRK4 S143T).

2. A vector according to claim 1 wherein said vector is a virus, chosen from an adeno-associated virus (AAV), an adenovirus, a lentivirus, an SV40 viral vector.

3. A vector according to claim 1 or 2, wherein the vector is an AAV2 or AAV9 virus comprising a 7 to 11 amino acid long insertion peptide in the GH loop of the VP1 capsid protein, wherein the insertion peptide comprises amino acid sequence LGETTRP (SEQ ID NO: 7).

4. A vector according to any one of claims 1 to 3, wherein the nucleotide sequence encoding the S143T mutated form of GIRK4 (GIRK4 S143T) is under the control of a cone-specific promoter.

5. A vector according to claim 4, wherein the cone-specific promoter is pR1.7 or a functional variant thereof, or minimal M-opsin promoter, in particular in a pMNTC expression cassette, or a GRK promoter or truncated version thereof.

6. The vector according to any one of claims 1 to 4, wherein the nucleotide sequence encoding GIRK4 S143T comprises the sequence SEQ ID NO: 3.

7. The vector according to any of the above claims, wherein the vector is a recombinant AAV9 vector comprising:
- a VP1 capsid protein in a 7 to 11 amino acid long insertion peptide is inserted in the GH loop of said VP1 capsid protein relative to wild-type AAV9 VP1 capsid protein, at a position localized between amino acids 588 and 589 of wild-type AAV9 VP1 capsid protein, wherein said peptide comprises amino acid sequence LGETTRP (SEQ ID NO: 5); and
- the nucleotide sequence encoding GIRK4 S143T under the control of a pR1.7 promoter.

8. The vector according to any of the above claims, wherein said insertion peptide comprises or consists of amino acid sequence AALGETTRPA (SEQ ID NO: 10), LALGETTRPA (SEQ ID NO: 11), or GLGETTRPA (SEQ ID NO: 12).

9. The vector according to any of the above claims, further comprising a nucleotide sequence encoding a mammalian cone opsin.

10. A pharmaceutically acceptable carrier comprising the vector defined in any one of claims 1 to 9.

11. The pharmaceutically acceptable carrier according to claim 10, which further comprises a vector comprising a nucleotide sequence encoding a mammalian cone opsin.

12. The pharmaceutically acceptable carrier according to claim 10 wherein the vector comprising a nucleotide sequence encoding a mammalian cone opsin:
a) is selected from the group consisting of an adeno-associated virus (AAV), an adenovirus, a lentivirus, and SV40 viral vector; or
b) is an AAV2 or AAV9 virus comprising a 7 to 11 amino acid long insertion peptide in the GH loop of the VP1 capsid protein, wherein the insertion peptide comprises amino acid sequence LGETTRP (SEQ ID NO: 7);or
c) is a recombinant AAV9 vector comprising:
- a VP1 capsid protein in a 7 to 11 amino acid long insertion peptide is inserted in the GH loop of said VP1 capsid protein relative to wild-type AAV9 VP1 capsid protein, at a position localized between amino acids 588 and 589 of wild-type AAV9 VP1 capsid protein, wherein said peptide comprises amino acid sequence LGETTRP (SEQ ID NO: 7); and
- the nucleotide sequence encoding the mammalian cone opsin under the control of a pR1.7 promoter.

13. The pharmaceutically acceptable carrier according to any one of claims 10 to 12 wherein the carrier is chosen from the group consisting of solid-lipid nanoparticles, chitosan nanoparticles, liposome, lipoplex and cationic polymer.

14. The vector according to any one of claims 1 to 9 or pharmaceutically acceptable carrier according to any one of claims 10 to 13, wherein the mammalian cone opsin is a short wavelength cone opsin (SWO).

15. A pharmaceutical composition comprising the vector according to any one of claims 1 to 9 and 14, or the pharmaceutically acceptable carrier according to any one of claims 10 to 14, with a pharmaceutically acceptable carrier, diluent or excipient.

16. The pharmaceutical composition according to claim 15, further comprising a vector comprising a nucleotide sequence encoding a mammalian cone opsin.

17. The vector according to any one of claims 1 to 9 and 14, the pharmaceutically acceptable carrier according to any one of claims 10 to 14, or the pharmaceutical composition according to claim 15 or 16, for use in treating retinal degenerative disease such as rod-cone dystrophy or cone-rod dystrophy.

18. The vector, pharmaceutically acceptable carrier or pharmaceutical composition for the use according to claim 17, wherein the vector, pharmaceutically acceptable carrier or pharmaceutical composition is administered by subretinal injection at distance of the fovea.

19. The vector, pharmaceutically acceptable carrier or pharmaceutical composition for the use according to any of claims 17 or 18 wherein the vector, pharmaceutically acceptable carrier or pharmaceutical composition is administered by subretinal injection a) in a region adjacent to the superior or inferior temporal branch of retinal artery; b) at a distance of 2-3 optic disk diameter away from the center of the fovea; and c) at a position localized in the geometric shape delineated by the branches of temporal retinal artery and temporal retinal vein.

20. The vector, pharmaceutically acceptable carrier or pharmaceutical composition for the use according to claim 17, wherein the vector, pharmaceutically acceptable carrier or pharmaceutical composition is administered by intravitreal injection.

21. A nucleic acid comprising a sequence encoding a S143T mutated form of the subunit 4 of G-protein-gated inwardly rectifying potassium channel (GIRK4) (GIRK4 S143T) for use in treating retinal degenerative disease such as rod-cone dystrophy or cone-rod dystrophy.
